# EUROPEAN PATENT SPECIFICATION

(11) **EP 0 689 436 B1**
(45) Date of publication and mention of the grant of the patent: **26.11.2003**
(21) Application number: 94910982.1
(22) Date of filing: 21.03.1994
(51) Int. Cl.: A61K 31/335, C07D 305/14, C07D 205/08, C07D 205/085, C07D 407/12, C07D 409/12, A61P 35/02

(54) **TAXANES NOT HAVING AN OXO GROUP AT C-9 AND PHARMACEUTICAL COMPOSITIONS CONTAINING SAME**
TAXANE OHNE DIE C-9 OXO GRUPPE UND DIESE ENTHALTENDE PHARMAZEUTISCHE PRÄPARATE
TAXANES NE PAS PRESENTANT UN GROUPE C-9 OXO ET COMPOSITIONS PHARMACEUTIQUES CONTENANT DE TELS COMPOSES

(30) Priority: 22.03.1993 US 34852; 20.07.1993 US 94717
(43) Date of publication of application: 03.01.1996
(73) Proprietor: FLORIDA STATE UNIVERSITY, Tallahassee, Florida 32310 (US)
(72) Inventor: HOLTON, Robert,A., 2035 East Paul Dirac Drive, Tallahassee, FL 32310 (US); NADIZADEH, Hossain, 2035 East Paul Dirac Drive, Tallahassee, FL 32310 (US); CHAI, Ki-byung, 2035 East Paul Dirac Drive, Tallahassee, FL 32310 (US); RENGAN, Kasthuri, 2035 East Paul Dirac Drive, Tallahassee, FL 32310 (US); SUZUKI, Yukio, 2035 East Paul Dirac Drive, Tallahassee, FL 32310 (US); TAO, Chunlin, 2035 East Paul Dirac Drive, Tallahassee, FL 32310 (US); IDMOUMAZ, Hamid, 2035 East Paul Dirac Drive, Tallahassee, FL 32310 (US)
(74) Representative: Eyles, Christopher Thomas
(86) International application number: US9403053
(87) International publication number: WO94021250

(56) References cited:
- US-A- 5 175 315
- US-A- 5 227 400

## Description

### BACKGROUND OF THE INVENTION

The present invention is directed to novel taxanes which have utility as antileukemia and antitumor agents.

The taxane family of terpenes, of which taxol is a member, has attracted considerable interest in both the biological and chemical arts. Taxol is a promising cancer chemotherapeutic agent with a broad spectrum of antileukemic and tumor-inhibiting activity. Taxol has a 2'R, 3'S configuration and the following structural formula: 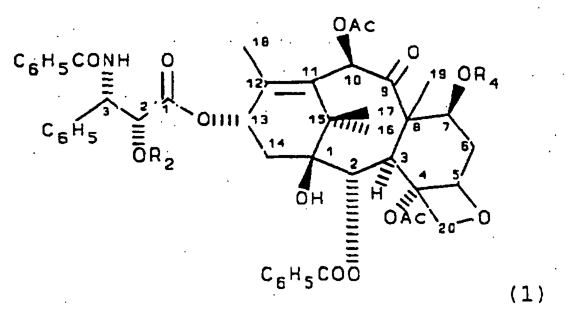 wherein Ac is acetyl. Because of this promising activity, taxol is currently undergoing clinical trials in both France and the United States.

Colin et al. reported in U.S. Patent No. 4,814,470 that taxol derivatives having structural formula **(2)** below, have an activity significantly greater than that of taxol **(1)**. 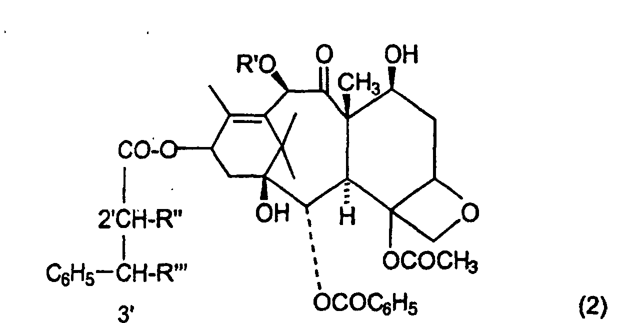 R' represents hydrogen or acetyl and one of R" and R''' represents hydroxy and the other represents tert-butoxycarbonylamino and their stereoisomeric forms, and mixtures thereof. The compound of formula (2) in which R' is hydrogen, R" is hydroxy, R''' is tert-butoxycarbonylamino having the 2'R, 3'S configuration is commonly referred to as taxotere.

Although taxol and taxotere are promising chemotherapeutic agents, they are not universally effective. Accordingly, a need remains for additional chemotherapeutic agents.

European Published Patent Specification No. 0 534 708 A, which forms part of the state of the art in relation to at least part of the subject matter claimed herein, discloses compounds of the general formula: 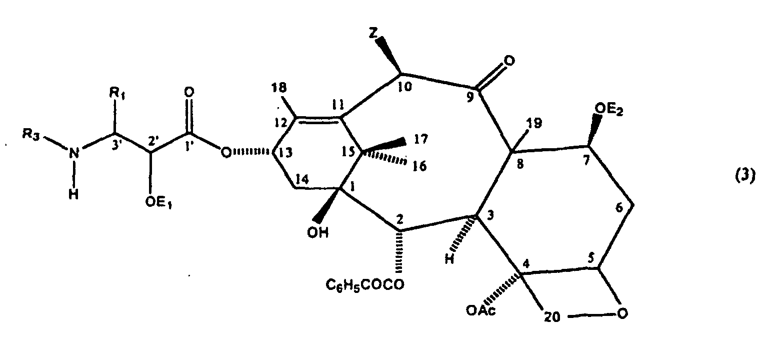 wherein
R₁ is

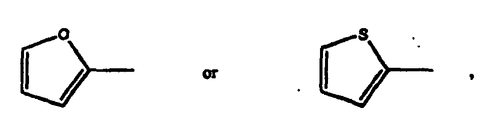

Z is -OT₁,
T₁ is hydrogen, hydroxyl protecting group, or -COT₂,
T₂ is H, C₁-C₆ alkenyl, C₁-C₆ alkynyl or monocyclic aryl,
R₃ is benzoyl, substituted benzoyl or C₁-C₆ alkoxycarbonyl,
Ac is acetyl, and
E₁ and E₂ are independently selected from hydrogen and functional groups which increase the water solubility of the taxane derivative.

### SUMMARY OF THE INVENTION

Among the objects of the present invention, therefore, is the provision of novel taxane derivatives which are valuable antileukemia and antitumor agents.

Briefly, therefore, the present invention is directed to taxane derivatives having a C13 side chain which includes an alkoxy, alkenoxy or aryloxy substituent, but which differs from taxotere with respect to at least one substituent. Such taxane derivatives have a tricyclic or tetracyclic core and correspond to the formula: 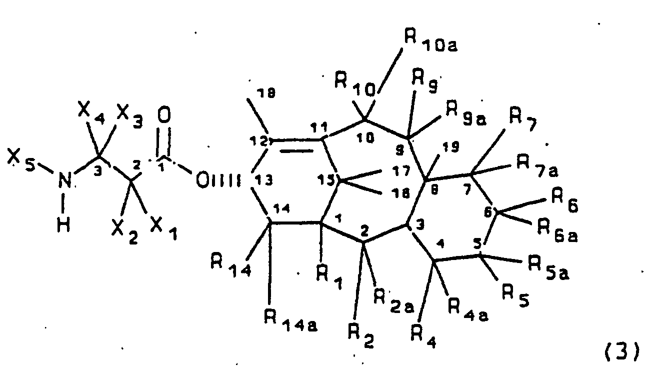 wherein
X₁ is -OX₆, -SX₇, or -NX₈X₉;
X₂ is hydrogen, alkyl, alkenyl, alkynyl, aryl, or heteroaryl;
X₃ and X₄ are independently hydrogen, alkyl, alkenyl, alkynyl, or heteroaryl;
X₅ is -COOX₁₀;
X₆ is hydrogen;
X₇ is alkyl, alkenyl, alkynyl, aryl, heteroaryl, or sulfhydryl protecting group;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is t-butyl;
R₁ is hydrogen, hydroxy, protected hydroxy or together with R₁₄ forms a carbonate;
R₂ is hydrogen, hydroxy, -OCOR₃₁ or together with R₂ₐ forms an oxo ;
R₂ₐ is hydrogen or taken together with R₂ forms an oxo or;
R₄ is hydrogen, together with R₄ₐ forms an oxo, oxirane or methylene, or together with R₅ₐ and the carbon atoms to which they are attached form an oxetane ring;
R₄ₐ is hydrogen, alkyl, alkenyl, alkynyl, aryl, heteroaryl, cyano, hydroxy, -OCOR₃₀, or together with R₄ forms an oxo, oxirane or methylene;
R₅ is hydrogen or together with R₅ₐ forms an oxo;
R₅ₐ is hydrogen, hydroxy, acyloxy, together with R₅ forms an oxo, or together with R₄ and the carbon atoms to which they are attached form an oxetane ring;
R₆ is hydrogen, alkyl, alkenyl, alkynyl, aryl, or heteroaryl, hydroxy, or together with R₆ₐ forms an oxo;
R₆ₐ is hydrogen, alkyl, alkenyl, alkynyl, aryl, or heteroaryl, hydroxy, or together with R₆ forms an oxo;
R₇ is hydrogen or together with R₇ₐ forms an oxo;
R₇ₐ is hydroxy, or together with R₇ forms an oxo;
R₉ is hydrogen;
R₉ₐ is hydrogen, hydroxy, or acyloxy;
R₁₀ is hydrogen or together with R₁₀ₐ forms an oxo ;
R₁₀ₐ is hydrogen, -OCOR₂₉, or hydroxy, or together with R₁₀ forms an oxo;
R₁₄ is hydrogen, alkyl, alkenyl, alkynyl, aryl, or heteroaryl, hydroxy, or together with R₁ forms a carbonate;
R₁₄ₐ is hydrogen, alkyl, alkenyl, alkynyl, aryl, or heteroaryl;
R₂₉, R₃₀, and R₃₁ are independently hydrogen, alkyl, alkenyl, alkynyl, monocyclic aryl or monocyclic heteroaryl,

Other objects and features of this invention will be in part apparent and in part pointed out hereinafter.

### DETAILED DESCRIPTION OF THE PREFERRED EMBODIMENTS

As used herein "Ar" means aryl; "Ph" means phenyl; "Ac" means acetyl; "Et" means ethyl; "R" means alkyl unless otherwise defined; "Bu" means butyl; "Pr" means propyl; "TES" means triethylsilyl; "TMS" means trimethylsilyl; "TPAP" means tetrapropylammonium perruthenate; "DMAP" means p-dimethylamino pyridine; "DMF" means dimethylformamide; "LDA" means lithium diisopropylamide; "LHMDS" means lithium hexamethyldisilazide; "LAH" means lithium aluminum hydride; "Red-Al" means sodium bis(2-methoxyethoxy) aluminum hydride; "AIBN" means azo-(bis)-isobutyronitrile; "10-DAB" means 10-desacetylbaccatin III; FAR means 2-chloro-1,1,2-trifluorotriethylamine; protected hydroxy means -OR wherein R is a hydroxy protecting group; sulfhydryl protecting group" includes, but is not limited to, hemithioacetals such as 1-ethoxyethyl and methoxymethyl, thioesters, or thiocarbonates; "amine protecting group" includes, but is not limited to, carbamates, for example, 2,2,2-trichloroethylcarbamate or tertbutylcarbamate; and "hydroxy protecting group" includes, but is not limited to, ethers such as methyl, t-butyl, benzyl, p-methoxybenzyl, p-nitrobenzyl, allyl, trityl, methoxymethyl, 2-methoxypropyl, methoxyethoxymethyl, ethoxyethyl, tetrahydropyranyl, tetrahydrothiopyranyl, and trialkylsilyl ethers such as trimethylsilyl ether, triethylsilyl ether, dimethylarylsilyl ether, triisopropylsilyl ether and t-butyldimethylsilyl ether; esters such as benzoyl, acetyl, phenylacetyl, formyl, mono-, di-, and trihaloacetyl such as chloroacetyl, dichloroacetyl, trichloroacetyl, trifluoroacetyl; and carbonates including but not limited to alkyl carbonates having from one to six carbon atoms such as methyl, ethyl, n-propyl, isopropyl, n-butyl, t-butyl; isobutyl, and n-pentyl; alkyl carbonates having from one to six carbon atoms and substituted with one or more halogen atoms such as 2,2,2-trichloroethoxymethyl and 2,2,2-trichloroethyl; alkenyl carbonates having from two to six carbon atoms such as vinyl and allyl; cycloalkyl carbonates having from three to six carbon atoms such as cyclopropyl, cyclobutyl, cyclopentyl and cyclohexyl; and phenyl or benzyl carbonates optionally substituted on the ring with one or more C₁₋₆ alkoxy, or nitro. Other hydroxyl, sulfhydryl and amine protecting groups may be found in "Protective Groups in Organic Synthesis" by T. W. Greene, John Wiley and Sons, 1981.

The alkyl groups described herein, either alone or with the various substituents defined herein are preferably lower alkyl containing from one to six carbon atoms in the principal chain and up to 15 carbon atoms. They may be substituted; straight, branched chain or cyclic and include methyl, ethyl, propyl, isopropyl, butyl, hexyl, cyclopropyl, cyclopentyl, and cyclohexyl.

The alkenyl groups described herein, either alone or with the various substituents defined herein are preferably lower alkenyl containing from two to six carbon atoms in the principal chain and up to 15 carbon atoms. They may be substituted, straight or branched chain and include ethenyl, propenyl, isopropenyl, butenyl, isobutenyl, hexenyl.

The alkynyl groups described herein, either alone or with the various substituents defined herein are preferably lower alkynyl containing from two to six carbon atoms in the principal chain and up to 15 carbon atoms. They may be substituted, straight or branched chain and include ethynyl, propynyl, butynyl, isobutynyl, and hexynyl.

The aryl moieties described herein, either alone or with various substituents, contain from 6 to 15 carbon atoms and include phenyl. Substituents include alkanoxy, protected hydroxy, halogen, alkyl, aryl, alkenyl, acyl, acyloxy, nitro, amino, amido, etc. Phenyl is the more preferred aryl.

The heteroaryl moieties described herein, either alone or with various substituents, contain from 5 to 15 atoms and include, furyl, thienyl, pyridyl and the like. Substituents include alkanoxy, protected hydroxy, halogen, alkyl, aryl, alkenyl, acyl, acyloxy, nitro, amino, and amido.

The acyloxy groups described herein contain alkyl, alkenyl, alkynyl, aryl or heteroaryl groups.

The substituents of the substituted alkyl, alkenyl, alkynyl, aryl, and heteroaryl groups and moieties described herein, may be alkyl, alkenyl, alkynyl, aryl, heteroaryl and/or may contain nitrogen, oxygen, sulfur, halogens and include, for example, lower alkoxy such as methoxy, ethoxy, butoxy, halogen such as chloro or fluoro, nitro, amino, and keto.

In accordance with the present invention, it has been discovered that compounds corresponding to structural formula **3** show remarkable properties, in vitro, and are valuable antileukemia and antitumor agents. Their biological activity has been determined in vitro, using tubulin assays according to the method of Parness et al., J. Cell Biology, 91: 479-487 (1981) and human cancer cell lines, and is comparable to that exhibited by taxol and taxotere.

In the taxane derivatives of the present invention, the taxane has a structure which differs from that of taxotere with respect to the C13 side chain and at least one other substituent. For example, R₁₄ may be hydroxy, R₂ may be hydroxy or -OCOR₃₁ wherein R₃₁ is hydrogen, alkyl or selected from the group comprising 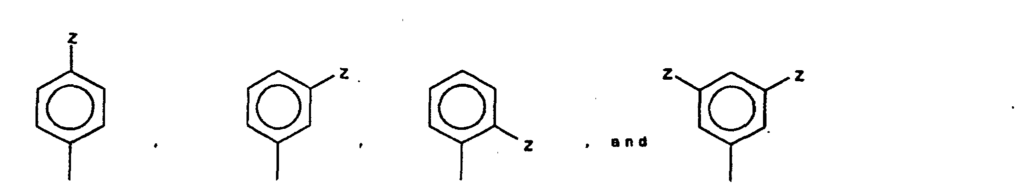 and Z is alkyl, hydroxy, alkoxy, halogen, or trifluoro methyl. R₉ is hydrogen and R₉ₐ may be hydrogen or hydroxy, R₇ may be hydrogen and R₇ₐ may be acetoxy or other acyloxy, or R₁₀ and R₁₀ₐ may each be hydrogen or together form an oxo.

With respect to the C13 side-chain, in a preferred embodiment X₁ is -OH, X₂ is hydrogen, X₃ is alkyl, alkenyl, phenyl or heteroaryl, X₄ is hydrogen, X₅ is -COOX₁₀, and X₁₀ is C₃ to C₈ alkyl, alkenyl or aryl and the taxane has the 2'R, 3'S configuration. In a particularly preferred embodiment, X₃ is isopropyl, cyclopropyl, n-butyl, t-butyl, cyclobutyl, cyclohexyl, furyl, thienyl, pyridyl or the substituted analogs thereof, X₅ is -COOX₁₀ and X₁₀ is tert-butyl.

Significantly, however, at least one of R₁, R₂, R₂ₐ, R₄, R₄ₐ, R₅, R₅ₐ, R₆, R₇, R₇ₐ, R₉, R₉ₐ, R₁₄, or R₁₄ₐ has a value such that the structure of the taxane is different from that of taxotere. For example, R₉ and R₉ₐ together do not form an oxo; and at least one of the following conditions may exist:
X₁ is -OX₆, -SX₇, or -NX₈X₉ and X₆ is other than hydrogen or hydroxy protecting group;
X₂ is other than hydrogen;
R₁ is other than hydroxy, e.g., together with R₁₄ it forms a carbonate;
R₂ is other than benzoylozy;
R₄ and R₅ₐ and the carbon atoms to which they are attached do not form an oxetane ring;
R₄ₐ is other than acetoxy;
R₆ and R₆ₐ are other than hydrogen;
R₇ₐ is other than hydroxy;
R₁₀ₐ is other than hydroxy; or
R₁₄ and R₁₄ₐ are other than hydrogen.

Taxanes having the general formula **3** may be obtained by reacting a β-lactam with metal alkoxides having the taxane tricyclic or tetracyclic nucleus and a C-13 metallic oxide substituent to form compounds having a β-amido ester substituent at C-13. The β-lactams have the following structural formula: 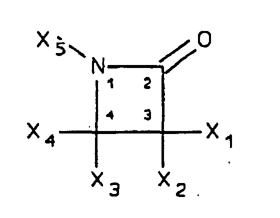 wherein X₁ - X₅ are as previously above.

The β-lactams can be prepared from readily available materials, as is illustrated in schemes A and B below: 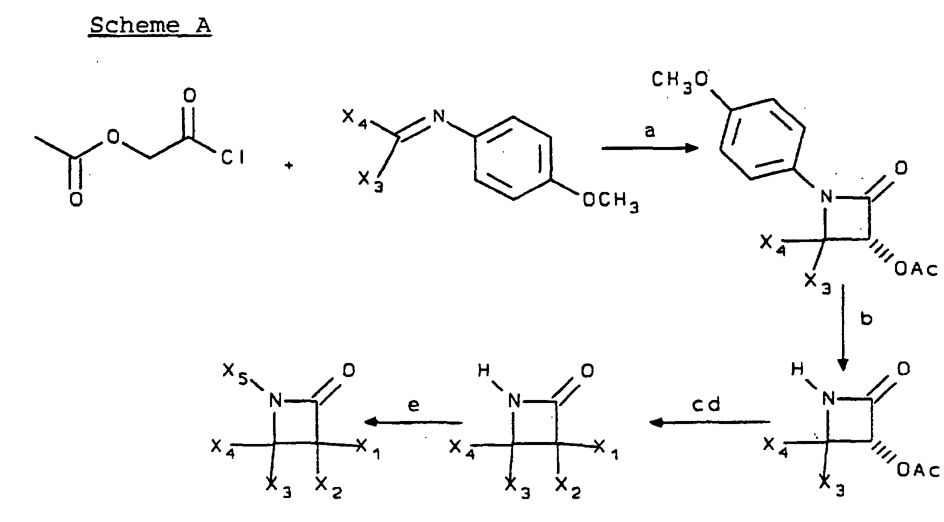 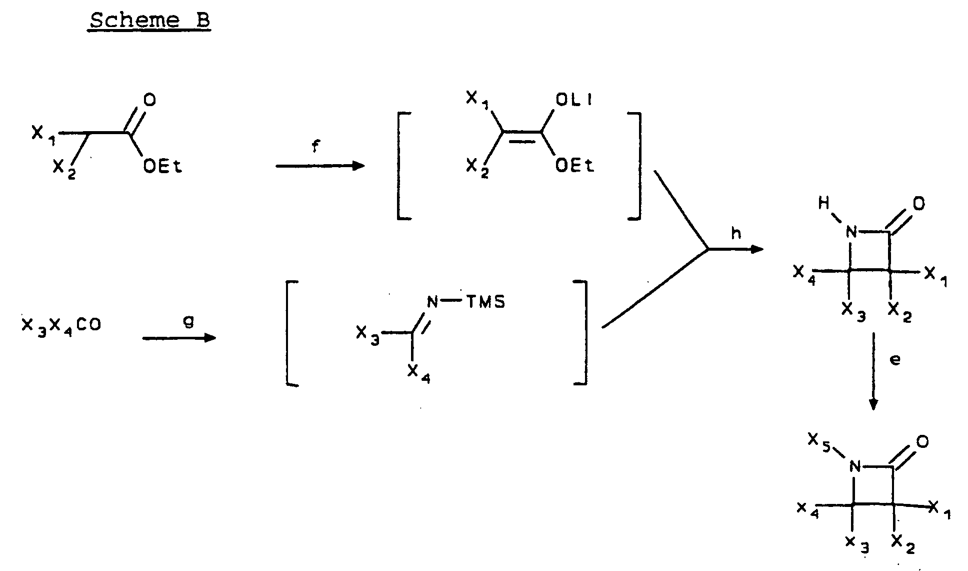 reagents: (a) triethylamine, CH₂Cl₂, 25°C, 18h; (b) 4 equiv ceric ammonium nitrate, CH₃CN, -10°C, 10 min; (c) KOH, THF, H₂O, 0°C, 30 min, or pyrolidine, pyridine, 25 °C, 3h, (d) TESCl, pyridine, 25 °C, 30 min or 2-methoxypropene toluene sulfonic acid (cat.), THF, 0°C, 2h; (e) n-butyllithium, THF, -78 °C, 30 min; and an acyl chloride or chloroformate (X₅ = -COX₁₀), sulfonyl chloride (X₅ = -COSX₁₀) or isocyanate (X₅ = -CONX₈X₁₀); (f) lithium diisopropyl amide, THF -78°C to -50°C; (g) lithium hexamethyldisilazide, THF -78°C to 0°C; (h) THF, -78°C to 25°C, 12h.

The starting materials are readily available. In scheme A, α-acetoxy acetyl chloride is prepared from glycolic acid, and, in the presence of a tertiary amine, it cyclocondenses with imines prepared from aldehydes and p-methoxyaniline to give 1-p-methoxyphenyl-3-acyloxy-4-arylazetidin-2-ones. The p-methoxyphenyl group can be readily removed through oxidation with ceric ammonium nitrate, and the acyloxy group can be hydrolyzed under standard conditions familiar to those experienced in the art to provide 3-hydroxy-4-arylazetidin-2-ones. In Scheme B, ethyl-α-triethylsilyloxyacetate is readily prepared from glycolic acid.

In Schemes A and B, X₁ is preferably -OX₆ and X₆ is a hydroxy protecting group. Protecting groups such as 2-methoxypropyl ("MOP"), 1-ethoxyethyl ("EE") are preferred, but a variety of other standard protecting groups such as the triethylsilyl group or other trialkyl (or aryl) silyl groups may be used. As noted above, additional hydroxy protecting groups and the synthesis thereof may be found in "Protective groups in Organic Synthesis" by T.W. Greene, John Wiley & Sons, 1981.

The racemic β-lactams may be resolved into the pure enantiomers prior to protection by recrystallization of the corresponding 2-methoxy-2-(trifluoromethyl) phenylacetic esters. However, the reaction described hereinbelow in which the β-amido ester side chain is attached has the advantage of being highly diastereoselective, thus permitting the use of a racemic mixture of side chain precursor.

The alkoxides having the tricyclic or tetracyclic taxane nucleus and a C-13 metallic oxide or ammonium oxide substituent which are useful in the synthesis of compounds of formula (3) have the following structural formula: 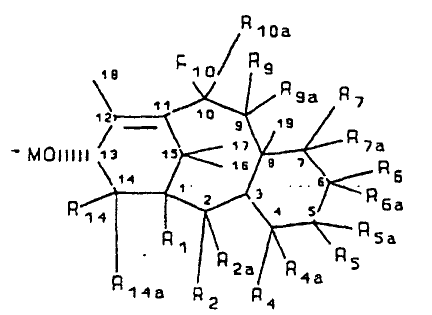 wherein R₁ - R₁₄ₐ are as previously defined and M comprises ammonium or is a metal optionally selected from the group comprising Group IA, Group IIA and transition metals, and preferably, Li, Mg, Na, K or Ti. Most preferably, the alkoxide has the tetracyclic taxane nucleus and corresponds to the structural formula: 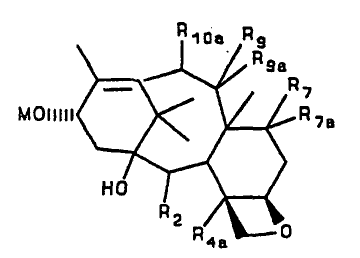 wherein M, R₂, R₄ₐ, R₇, R₇ₐ, R₉, R₉ₐ, R₁₀, and R₁₀ₐ are as previously defined.

The alkoxides can be prepared by reacting an alcohol having the taxane nucleus and a C-13 hydroxyl group with an organometallic compound in a suitable solvent. Most preferably, the alcohol is a protected baccatin III, in particular, 7-O-triethylsilyl baccatin III (which can be obtained as described by Greene, et al. in JACS 110: 5917 (1988) or by other routes) or 7,10-bis-O-triethylsilyl baccatin III.

As reported in Greene et al., 10-deacetyl baccatin III is converted to 7-O-triethylsilyl-10-deacetyl baccatin III according to the following reaction scheme: 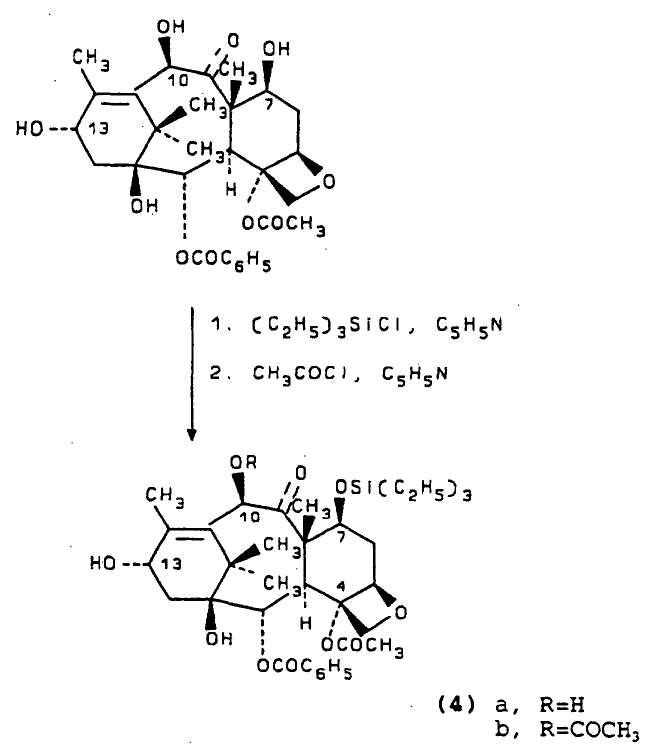 Under what is reported to be carefully optimized conditions, 10-deacetyl baccatin III is reacted with 20 equivalents of (C₂H₅)₃SiCl at 23°C under an argon atmosphere for 20 hours in the presence of 50 ml of pyridine/mmol of 10-deacetyl baccatin III to provide 7-triethylsilyl-10-deacetyl baccatin III **(4a)** as a reaction product in 84-86% yield after purification. The reaction product may then optionally be acetylated with 5 equivalents of CH₃COCl and 25 mL of pyridine/mmol of **4a** at 0 °C under an argon atmosphere for 48 hours to provide 86% yield of 7-O-triethylsilyl baccatin III **(4b)**. Greene, et al. in JACS 110, 5917 at 5918 (1988).

The 7-protected baccatin III **(4b)** is reacted with an organometallic compound such as LHMDS in a solvent such as tetrahydrofuran (THF), to form the metal alkoxide 13-O-lithium-7-O-triethylsilyl baccatin III as shown in the following reaction scheme:

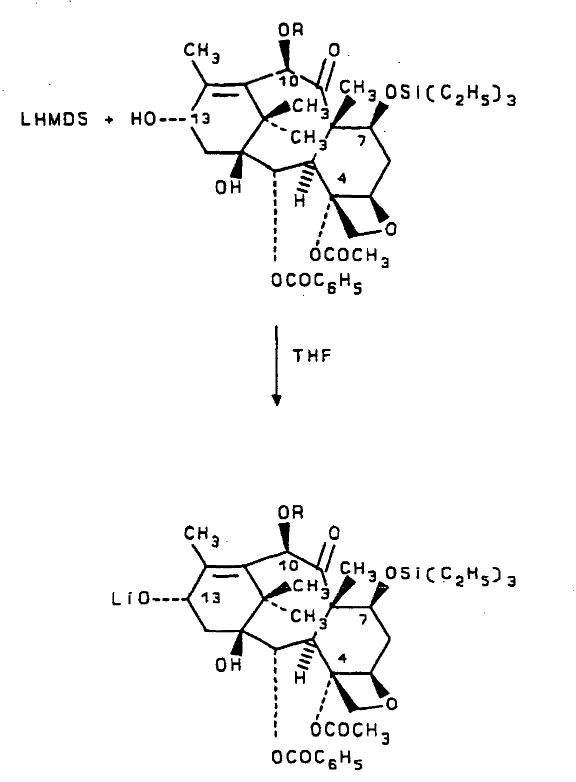

As shown in the following reaction scheme, 13-O-lithium-7-O-triethylsilyl baccatin III reacts with a β-lactam in which X₁ is preferably -OX₆, (X₆ being a hydroxy protecting group) and X₂ - X₅ are as previously defined to provide an intermediate in which the C-7 and C-2' hydroxyl groups are protected. The protecting groups are then hydrolyzed under mild conditions so as not to disturb the ester linkage or the taxane substituents.

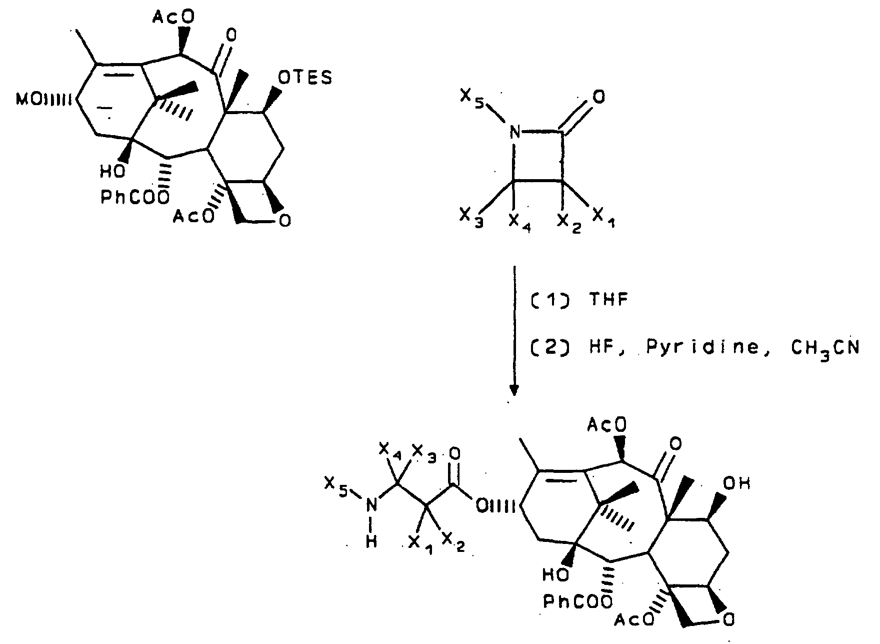

Both the conversion of the alcohol to the alkoxide and the ultimate synthesis of the taxane derivative can take place in the same reaction vessel. Preferably, the β-lactam is added to the reaction vessel after formation therein of the alkoxide.

Compounds of formula 3 of the instant invention are useful for inhibiting tumor growth in animals including humans and are preferably administered in the form of a pharmaceutical composition comprising an effective antitumor amount of compound of the instant invention in combination with a pharmaceutically acceptable carrier or diluent.

Antitumor compositions herein may be made up in any suitable form appropriate for desired use; e.g., oral, parenteral or topical administration. Examples of parenteral administration are intramuscular, intravenous, intraperitoneal, rectal and subcutaneous administration.

The diluent or carrier ingredients should not be such as to diminish the therapeutic effects of the antitumor compounds.

Suitable dosage forms for oral use include tablets, dispersible powders, granules, capsules, suspensions, syrups, and elixirs. Inert diluents and carriers for tablets include, for example, calcium carbonate, sodium carbonate, lactose and talc. Tablets may also contain granulating and disintegrating agents such as starch and alginic acid, binding agents such as starch, gelatin and acacia, and lubricating agents such as magnesium stearate, stearic acid and talc. Tablets may be uncoated or may be coated by unknown techniques; e.g., to delay disintegration and absorption. Inert diluents and carriers which may be used in capsules include, for example, calcium carbonate, calcium phosphate and kaolin. Suspensions, syrups and elixirs may contain conventional excipients, for example, methyl cellulose, tragacanth, sodium alginate; wetting agents, such as lecithin and polyoxyethylene stearate; and preservatives, e.g., ethyl- p-hydroxybenzoate.

Dosage forms suitable for parenteral administration include solutions, suspensions, dispersions, and emulsions. They may also be manufactured in the form of sterile solid compositions which can be dissolved or suspended in sterile injectable medium immediately before use. They may contain suspending or dispersing agents known in the art.

Taxanes having alternative C9 substituents may be prepared by selectively reducing the C9 keto substituent to yield the corresponding C9 β-hydroxy derivative. The reducing agent is preferably a borohydride and, most preferably, tetrabutylammoniumborohydride (Bu₄NBH₄) or triacetoxy-borohydride.

As illustrated in Reaction Scheme 1, the reaction of baccatin III with Bu₄NHH₄ in methylene chloride yields 9-desoxo-9β-hydroxybaccatin III **5**. After the C7 hydroxy group is protected with the triethylsilyl protecting group, for example, a suitable side chain may be attached to 7-protected-9β-hydroxy derivative **6** as elsewhere described herein. Removal of the remaining protecting groups thus yields 9β-hydroxy-desoxo taxol or other 9β-hydroxytetracyclic taxane having a C13 side chain.

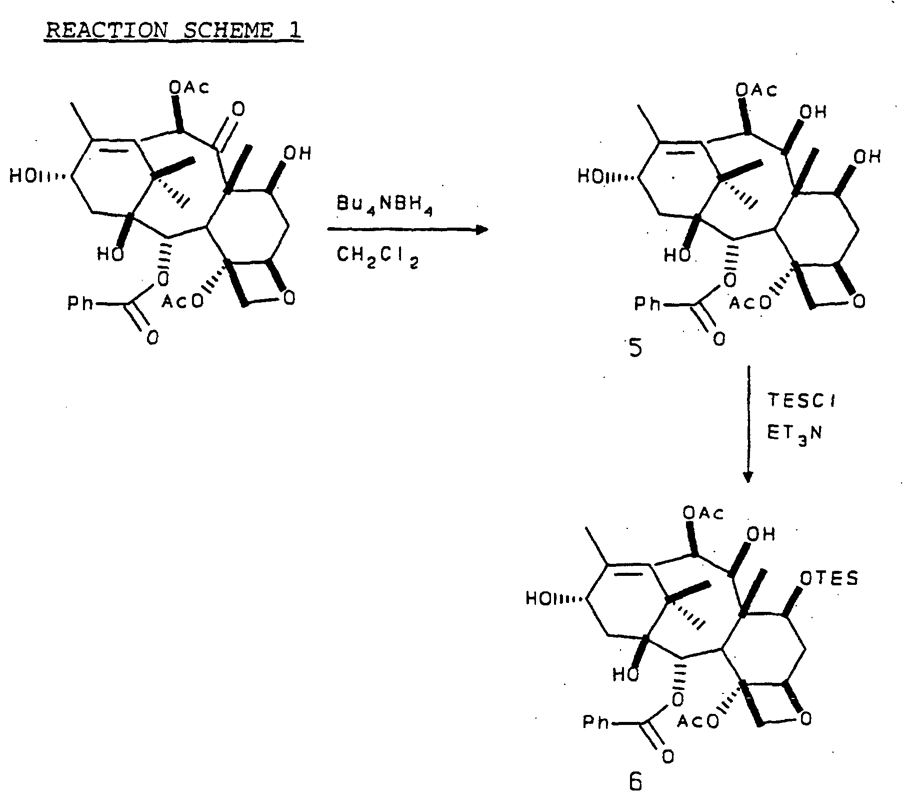

Alternatively, the C13 hydroxy group of 7-protected-9β-hydroxy derivative **6** may be protected with trimethylsilyl or other protecting group which can be selectively removed relative to the C7 hydroxy protecting group as illustrated in Reaction Scheme 2, to enable further selective manipulation of the various substituents of the taxane. For example, reaction of 7,13-protected-9β-hydroxy derivative **7** with KH causes the acetate group to migrate from C10 to C9 and the hydroxy group to migrate from C9 to C10, thereby yielding 10-desacetyl derivative **8**. Protection of the C10 hydroxy group of 10-desacetyl derivative **8** with triethylsilyl yields derivative **9**. Selective removal of the C13 hydroxy protecting group from derivative **9** yields derivative **10** to which a suitable side chain may be attached as described above.

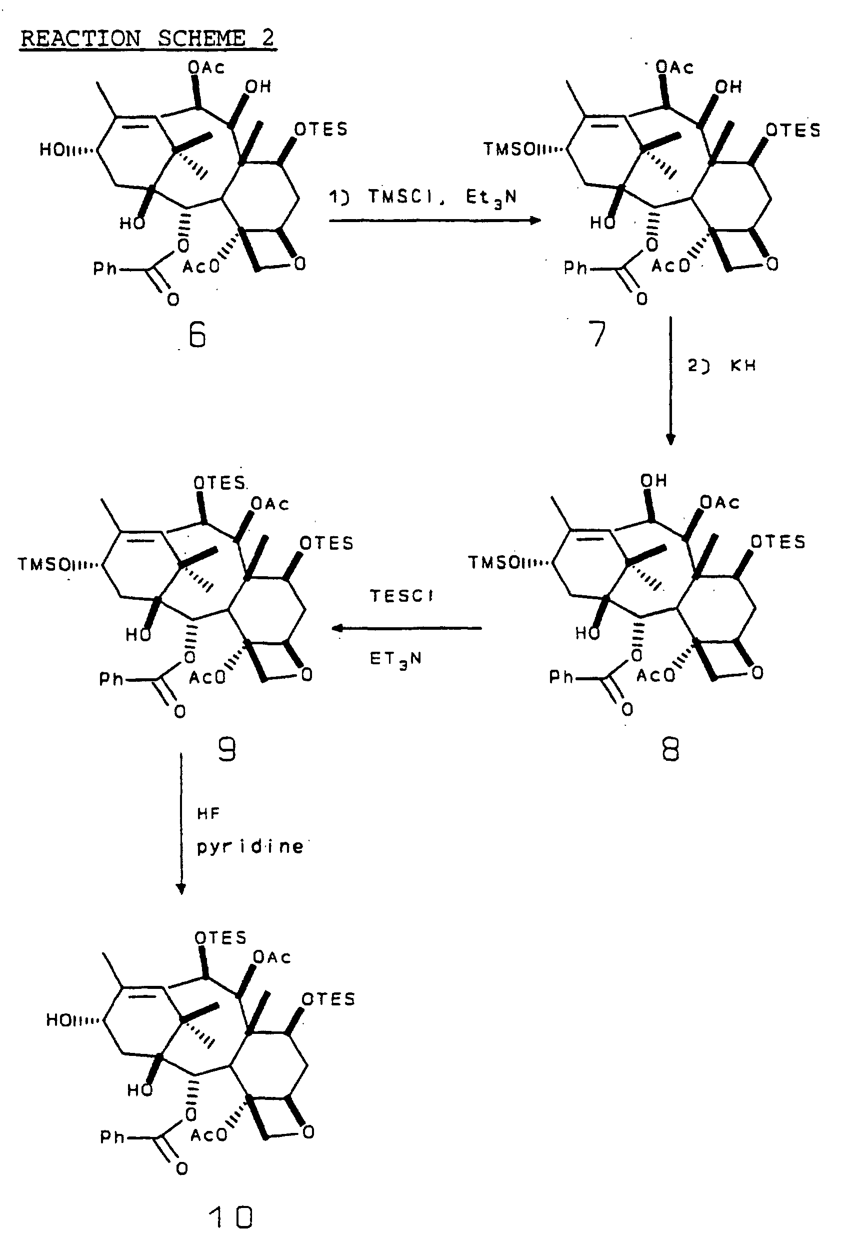

As shown in Reaction Scheme 3, 10-oxo derivative **11** can be provided by oxidation of 10-desacetyl derivative **8**. Thereafter, the C13 hydroxy protecting group can be selectively removed followed by attachment of a side chain as described above to yield 9-acetoxy-10-oxo-taxol or other 9-acetoxy-10-oxotetracylic taxanes having a C13 side chain. Alternatively, the C9 acetate group can be selectively removed by reduction of 10-oxo derivative **11** with a reducing agent such as samarium diiodide to yield 9-desoxo-10-oxo derivative **12** from which the C13 hydroxy protecting group can be selectively removed followed by attachment of a side chain as described above to yield 9-desoxo-10-oxo-taxol or other 9-desoxo-10-oxotetracyclic taxanes having a C13 side chain.

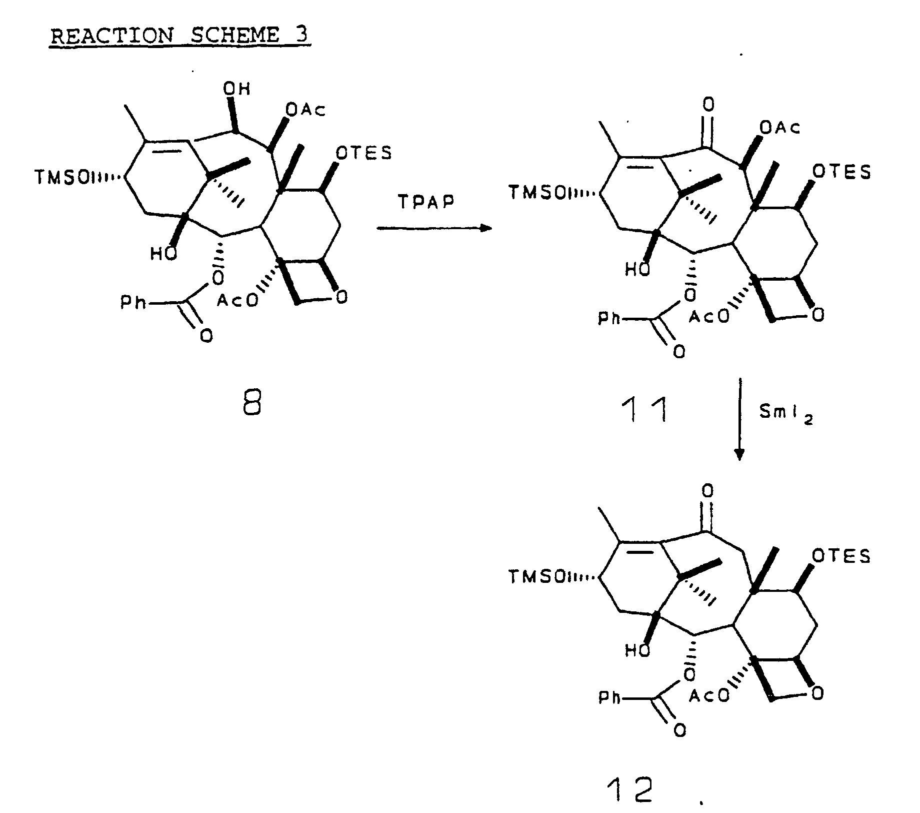

Reaction Scheme 4 illustrates a reaction in which 10-DAB is reduced to yield pentaol **13**. The C7 and C10 hydroxyl groups of pentaol **13** can then be selectively protected with the triethylsilyl or another protecting group to produce triol **14** to which a C13 side chain can be attached as described above or, alternatively, after further modification of the tetracyclic substituents.

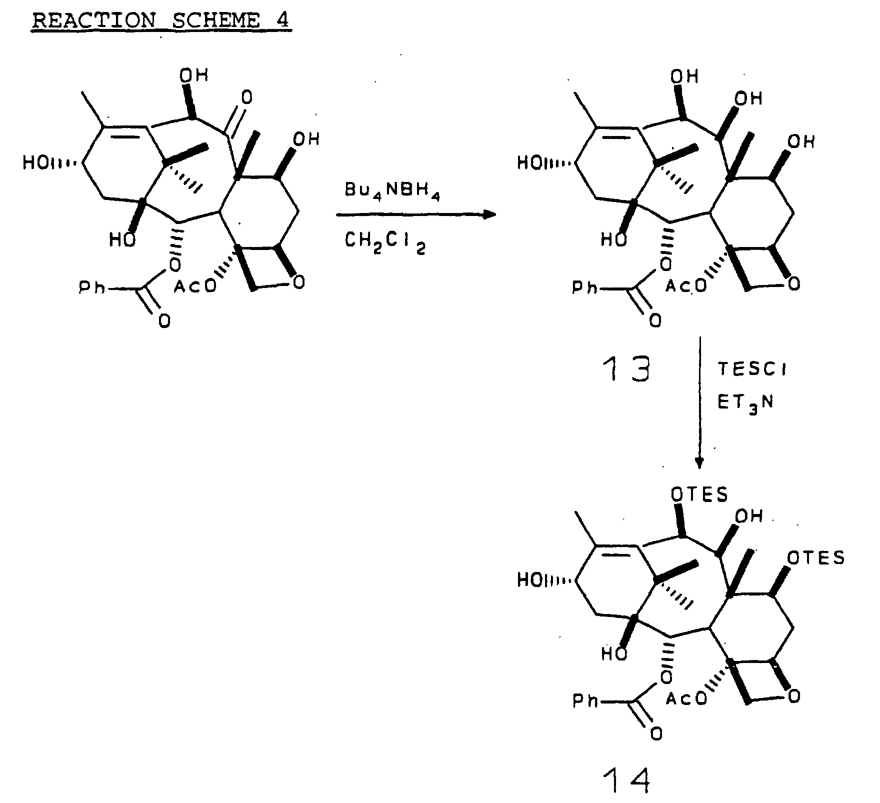

Taxanes having C9 and/or C10 acyloxy substituents other than acetate can be prepared using 10-DAB as a starting material as illustrated in Reaction Scheme 5. Reaction of 10-DAB with triethylsilyl chloride in pyridine yields 7-protected 10-DAB **15**. The C10 hydroxy substituent of 7-protected 10-DAB **15** may then be readily acylated with any standard acylating agent to yield derivative **16** having a new C10 acyloxy substituent. Selective reduction of the C9 keto substituent of derivative **16** yields 9β-hydroxy derivative **17** to which a C13 side chain may be attached. Alternatively, the C10 and C9 groups can be caused to migrate as set forth in Reaction Scheme **2**, above.

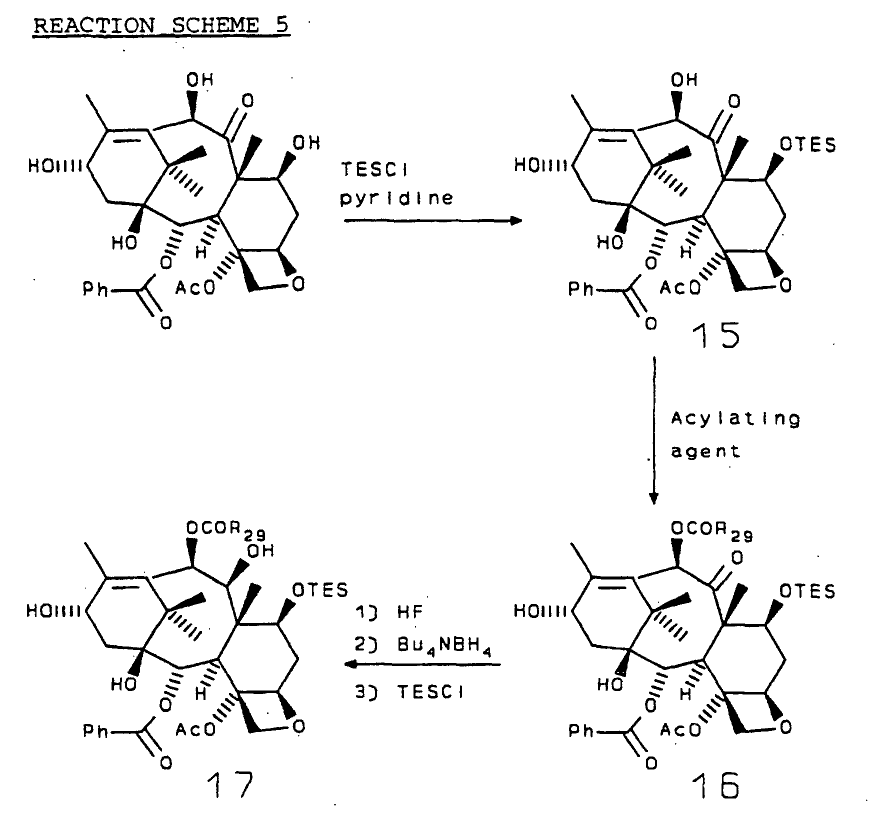

Taxanes having alternative C2 and/or C4 esters can be prepared using baccatin III and 10-DAB as starting materials. The C2 and/or C4 esters of baccatin III and 10-DAB can be selectively reduced to the corresponding alcohol(s) using reducing agents such as LAH or Red-Al, and new esters can thereafter be substituted using standard acylating agents such as anhydrides and acid chlorides in combination with an amine such as pyridine, triethylamine, DMAP, or diisopropyl ethyl amine. Alternatively, the C2 and/or C4 alcohols may be converted to new C2 and/or C4 esters through formation of the corresponding alkoxide by treatment of the alcohol with a suitable base such as LDA followed by an acylating agent such as an acid chloride.

Baccatin III and 10-DAB analogs having different substituents at C2 and/or C4 can be prepared as set forth in Reaction Schemes 6-10. To simplify the description, 10-DAB is used as the starting material. It should be understood, however, that baccatin III derivatives or analogs may be produced using the same series of reactions (except for the protection of the C10 hydroxy group) by simply replacing 10-DAB with baccatin III as the starting material. Derivatives of the baccatin III and 10-DAB analogs having different substituents at C10 and at least one other position, for instance C1, C2, C4, C7, C9 and C13, can then be prepared by carrying out any of the other reactions described herein and any others which are within the level of skill in the art.

In Reaction Scheme 6, protected 10-DAB **3** is converted to the triol **18** with lithium aluminum hydride. Triol **18** is then converted to the corresponding C4 ester using Cl₂CO in pyridine followed by a nucleophilic agent (e.g., Grignard reagents or alkyllithium reagents).

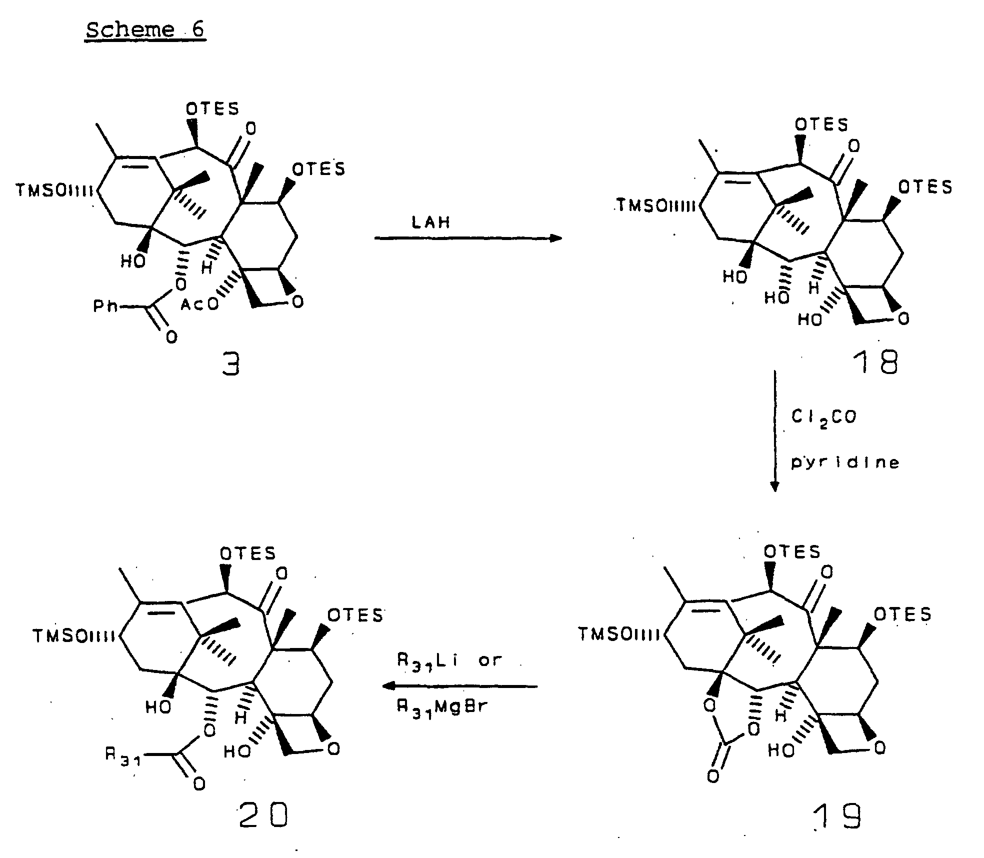

Deprotonation of triol **18** with LDA followed by introduction of an acid chloride selectively gives the C4 ester. For example, when acetyl chloride was used, triol **18** was converted to 1,2 diol **4** as set forth in Reaction Scheme 7.

Triol **18** can also readily be converted to the 1,2 carbonate **19.** Acetylation of carbonate **19** under vigorous standard conditions provides carbonate **21** as described in Reaction Scheme 8; addition of alkyllithiums or Grignard reagents to carbonate **19** provides the C2 ester having a free hydroxyl group at C4 as set forth in Reaction Scheme 6.

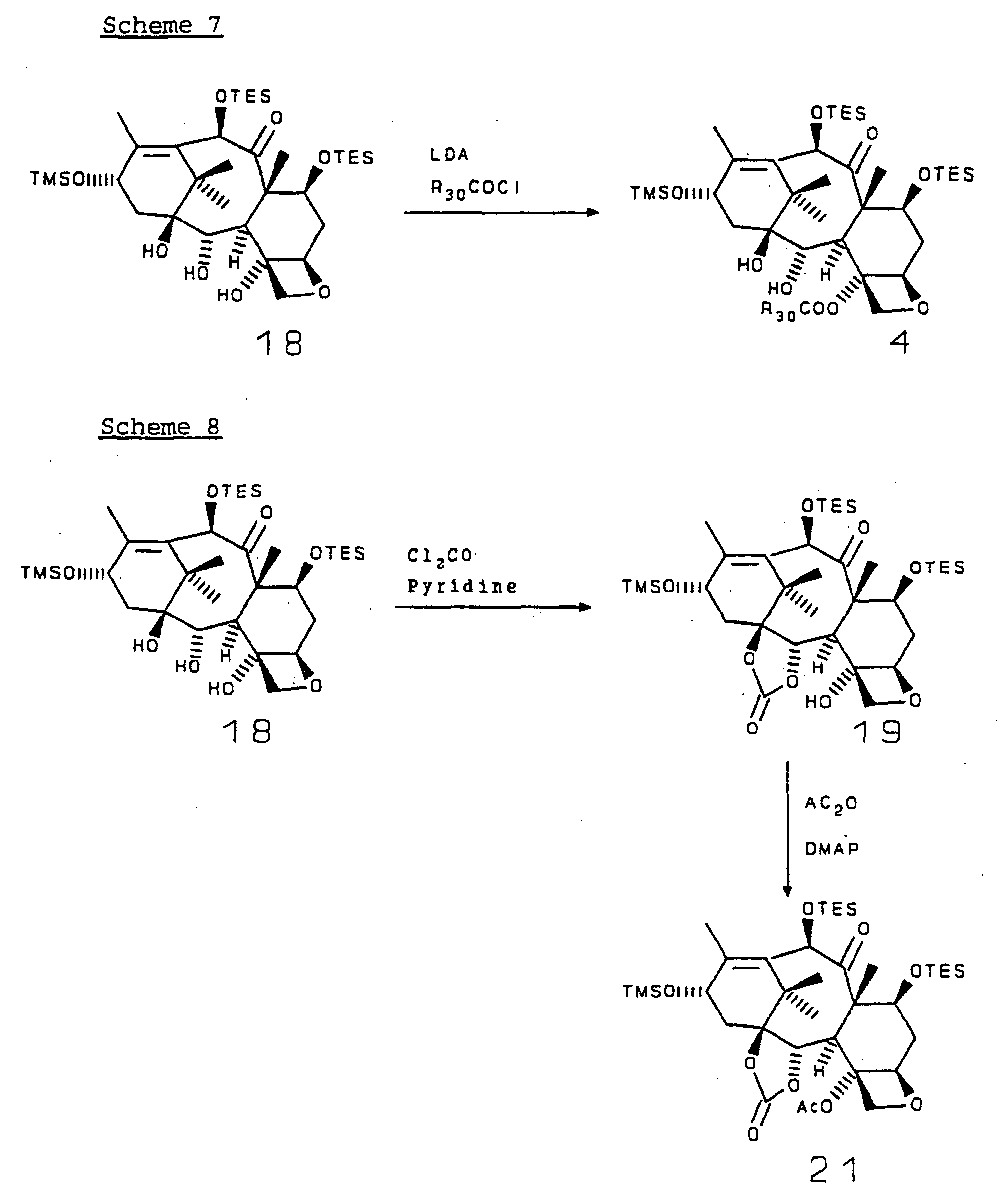

As set forth in Reaction Scheme 9, other C4 substituents can be provided by reacting carbonate **19** with an acid chloride and a tertiary amine to yield carbonate **22** which is then reacted with alkyllithiums or Grignard reagents to provide 10-DAB derivatives having new substituents at C2.

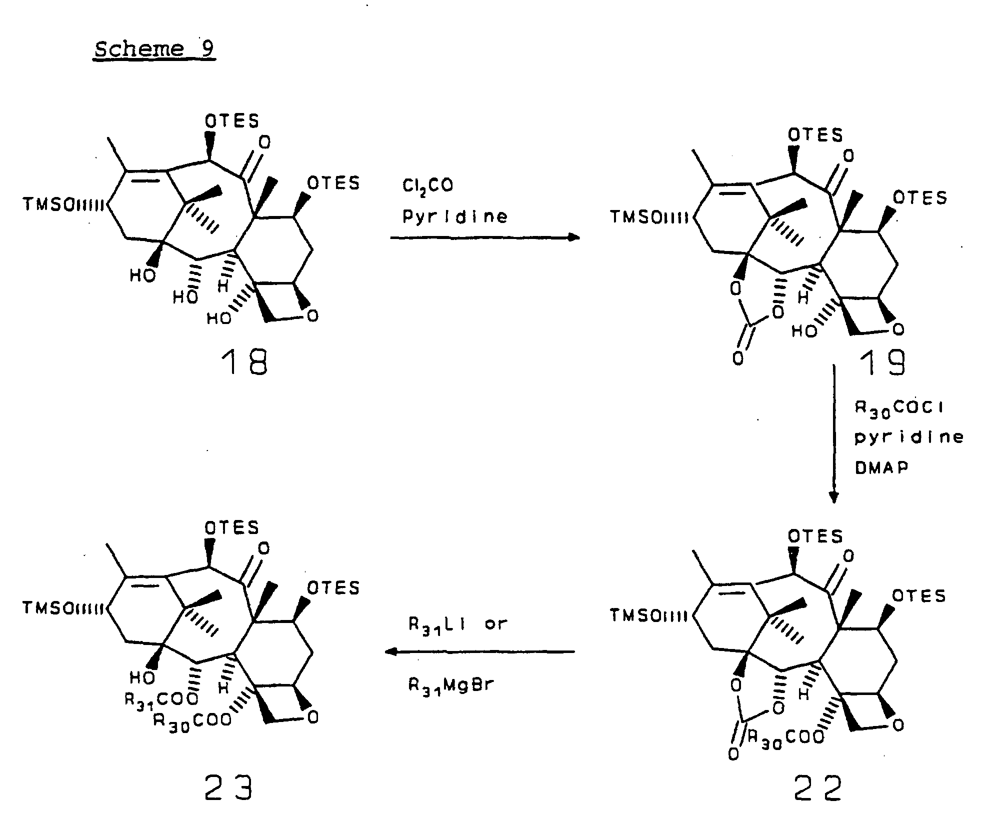

Alternatively, baccatin III may be used as a starting material and reacted as shown in Reaction Scheme 10. After being protected at C7 and C13, baccatin III is reduced with LAH to produce 1,2,4,10 tetraol **24**. Tetraol **24** is converted to carbonate **25** using Cl₂CO and pyridine, and carbonate **25** is acylated at C10 with an acid chloride and pyridine to produce carbonate **26** (as shown) or with acetic anhydride and pyridine (not shown). Acetylation of carbonate **26** under vigorous standard conditions provides carbonate **27** which is then reacted with alkyl lithiums to provide the baccatin III derivatives having new substituents at C2 and C10.

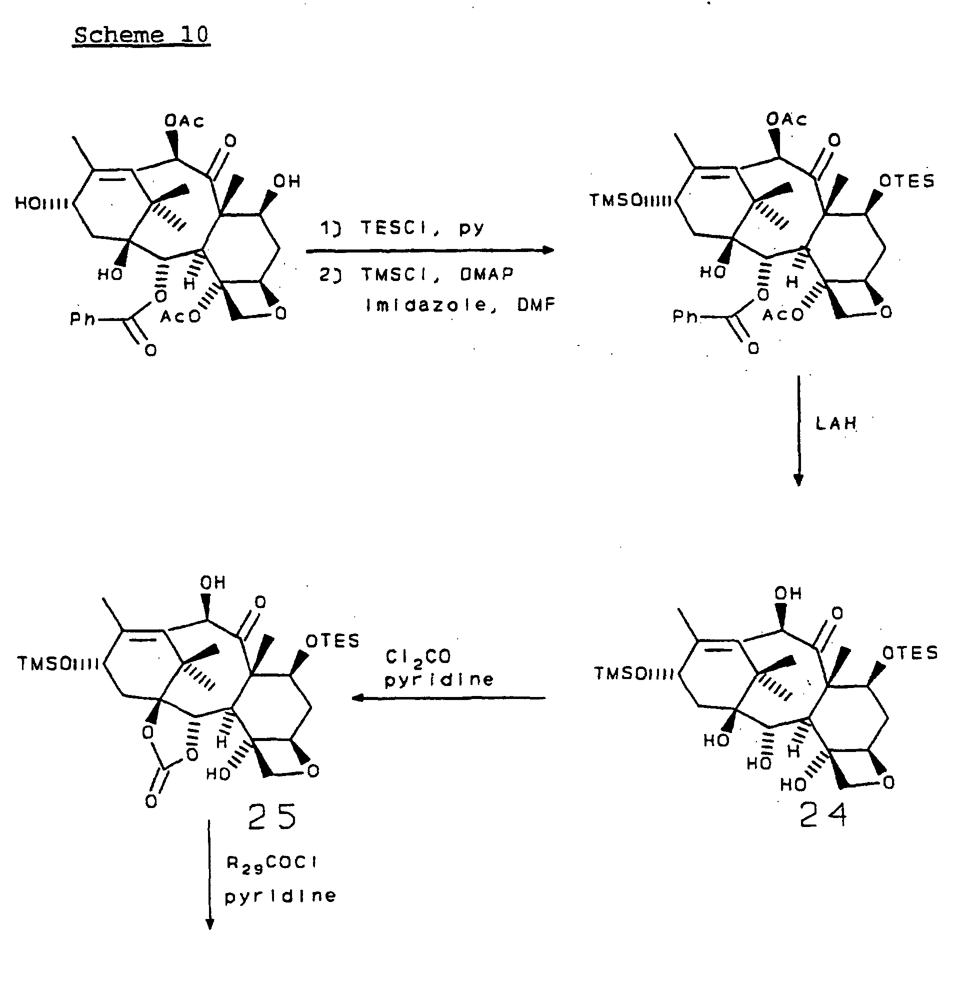

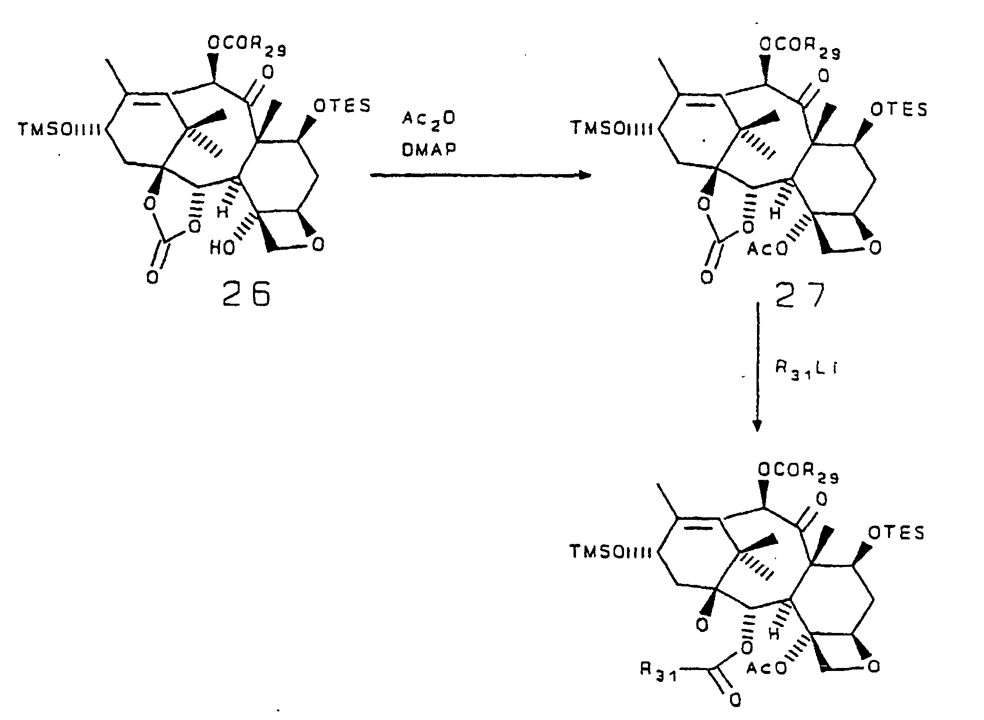

10-desacetoxy derivatives of baccatin III and 10-desoxy derivatives of 10-DAB may be prepared by reacting baccatin III or 10-DAB (or their derivatives) with samarium diiodide. Reaction between the tetracyclic taxane having a C10 leaving group and samarium diiodide may be carried out at 0°C in a solvent such as tetrahydrofuran. Advantageously, the samarium diiodide selectively abstracts the C10 leaving group; C13 side chains and other substituents on the tetracyclic nucleus remain undisturbed. Thereafter, the C9 keto substituent may be reduced to provide the corresponding 9-desoxo-9β-hydroxy-10-desacetoxy or 10-desoxy derivatives as otherwise described herein.

C7 substituted taxanes can be prepared as set forth in Reaction Scheme 11.

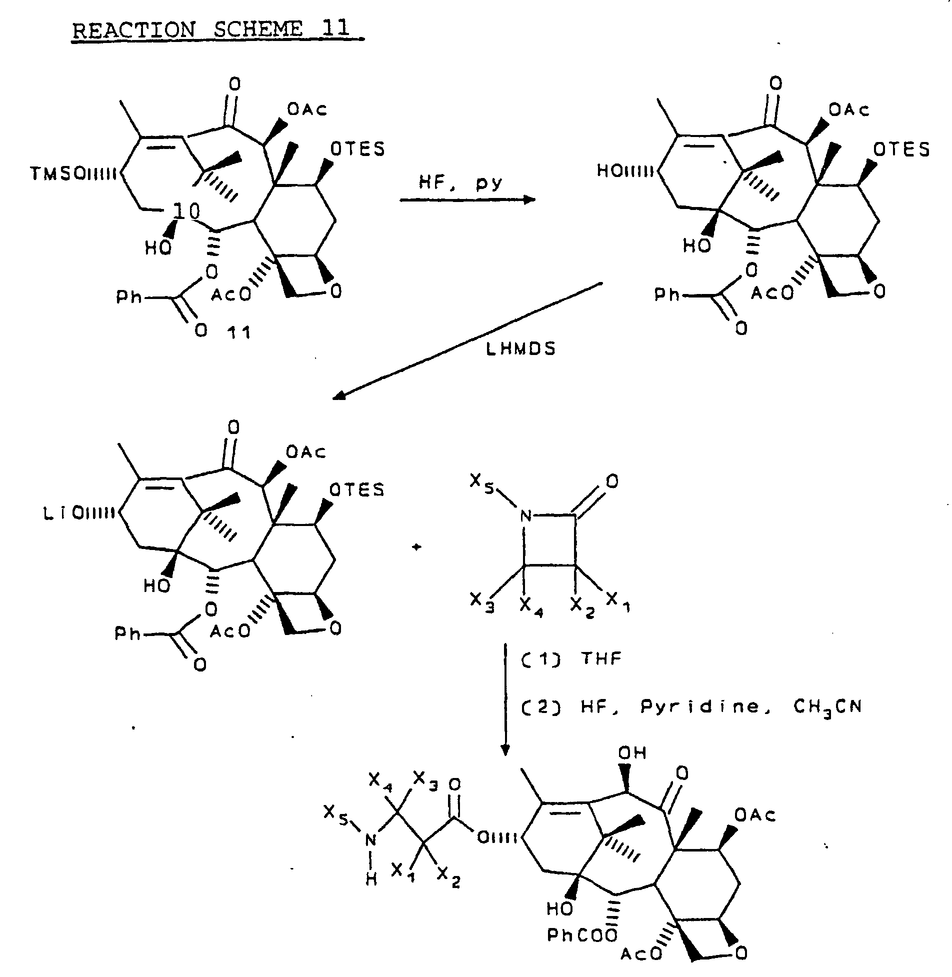

Taxanes having C7 acyloxy substituents can be prepared as set forth in Reaction Scheme 11, 7,13-protected 10-oxo-derivative 11 is converted to its corresponding C13 alkoxide by selectively removing the C13 protecting group and replacing it with a metal such as lithium. The.alkoxide is then reacted with a β-lactam or other side chain precursor. Subsequent hydrolysis of the C7 protecting groups causes a migration of the C7 hydroxy substituent to C10, migration of the C10 oxo substituent to C9, and migration of the C9 acyloxy substituent to C7.

A wide variety of tricyclic taxanes are naturally occurring, and through manipulations analogous to those described herein, an appropriate side chain can be attached to the C13 oxygen of these substances. Alternatively, as shown in Reaction Scheme 12, 7-O-triethylsilyl baccatin III can be converted to a tricyclic taxane through the action of trimethyloxonium tetrafluoroborate in methylene chloride solution. The product diol then reacts with lead tetraacetate to provide the corresponding C4 ketone.

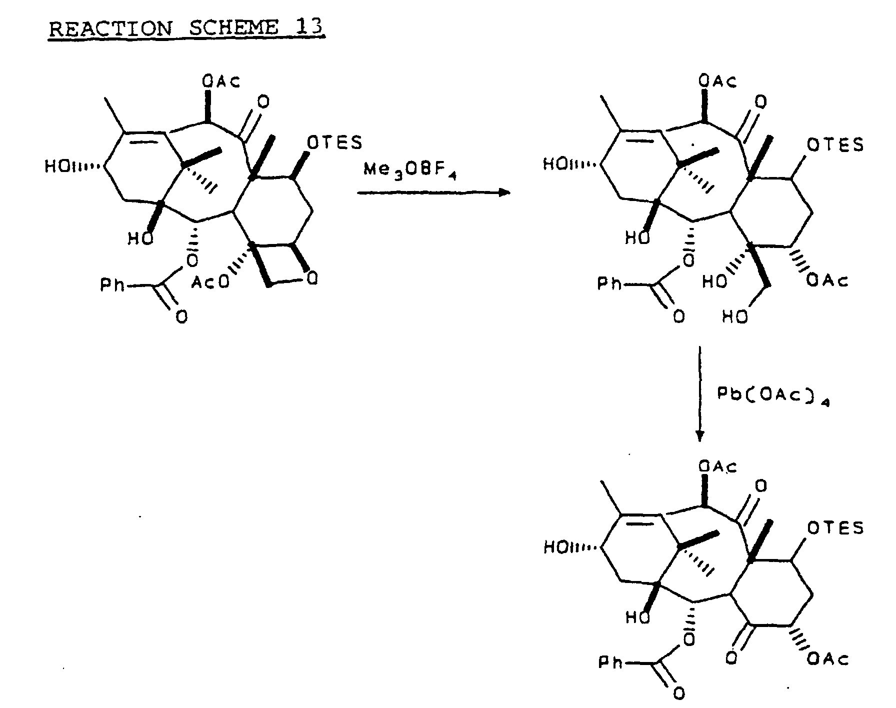

Recently a hydroxylated taxane (14-hydroxy-10-deacetylbaccatin III) has been discovered in an extract of yew needles (C&EN, p 36-37, April 12, 1993). Derivatives of this hydroxylated taxane having the various C2, C4, etc. functional groups described above may also be prepared by using this hydroxylated taxane. In addition, the C14 hydroxy group together with the C1 hydroxy group of 10-DAB can be converted to a 1,2-carbonate as described in C&EN or it may be converted to a variety of esters or other functional groups as otherwise described herein in connection with the C2, C4, C7, C9, C10 and C13 substituents.

The following Examples are provided to more fully illustrate the invention.

### EXAMPLE 1 COMPARATIVE)

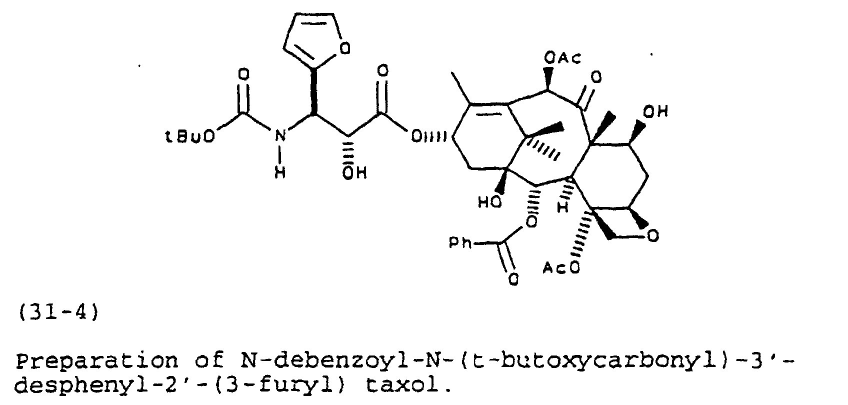

To a solution of 7-triethylsilyl baccatin III (120 mg, 0.171 mmol) in 1.2 mL of THF at -45 °C was added dropwise 0.104 mL of a 1.63M solution of nBuLi in hexane. After 0.5 h at -45 °C, a solution of cis-1-(t-butoxycarbonyl)-3-triethyl-silyloxy-4-(2-furyl)azetidin-2-one (314 mg, 0.885 mmol) in 1.2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 182 mg of a mixture containing (2'R,3'S)-2',7-(bis)-triethylsilyl-N-debenzoyl-N-(t-butoxycarbonyl)-3'-desphenyl-3'-(2-furyl) taxol and a very small amount of the (2'S,3'R) isomer.

To a solution of 182 mg (0.171 mmol) of the mixture obtained from the previous reaction in 11 mL of acetonitrile and 0.55 mL of pyridine at 0 °C was added 1.7 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 143 mg of material which was purified by recrystallization to give 133.0 mg (93%) of N-debenzoyl-N-(t-butoxycarbonyl)-3'-desphenyl-3'-(2-furyl) taxol.
m.p.155-156 °C; [α]²⁵_{Na}-73.0° (c 0.0065, CHCl₃).
¹H NMR (CDCl₃, 300 MHz) δ 8.11(d, J=7.1 Hz, 2H, benzoate ortho), 7.63-7.46 (m, 3H, aromatic), 7.41(d, J=1.1 Hz, 1H, furyl), 6.38-6.31(m, 2H, furyl), 6.30 (s, 1H, H10), 6.23 (dd, J = 9.4, 9.4 Hz, 1H, H13), 5.67(d, J = 7.1 Hz, 1H, H2β), 5.3(d, J=9.3 Hz, 1H, NH), 5.22(d, J=9.9 Hz, 1H, H3'), 4,95 (d, J = 9.3 Hz, 1H, H5), 4.71 (br s, 1H, H2'), 4.42 (m, 1H, H7), 4.30 (d, J=8.2 Hz, 1H, H20α), 4.16(d, J = 8.2 Hz, 1H, H20β), 3.82(d, J=7.1 Hz, 1H, H3), 3.29(d, J=5.5 Hz, 1H, 2'OH), 2.56 (m, 1H, H6α), 2.47 (d, J=3.6 Hz, 1H, 7OH), 2.39 (s, 3H, 4Ac), 2.34 (m, 2H, H14), 2.24 (s, 3H, 10Ac), 1.90(m, 1H, H6β), 1.88 (br s, 3H, Me18), 1.70 (s, 1H, 10H), 1.67 (s, 3H, Me19), 1.34(s, 9H, t-butyl), 1.25 (s, 3H, Me17), 1.14(s, 3H, Me16).

### EXAMPLES 2-6

Using the procedure set forth in Example 1 (except for the substituents of azetidin-2-one and the protected taxane and the amounts of the reactants) a series of compounds were prepared having the structure shown above in which X₃, X₁₀, R₂, R₇ₐ, R₉ₐ, and R₁₀ₐ are as shown in the following table. Unless otherwise indicated, R₂ is benzoyloxy, R₇ₐ is hydroxy, R₉ₐ is keto, and R₁₀ₐ is acetoxy. The structures were confirmed by NMR.

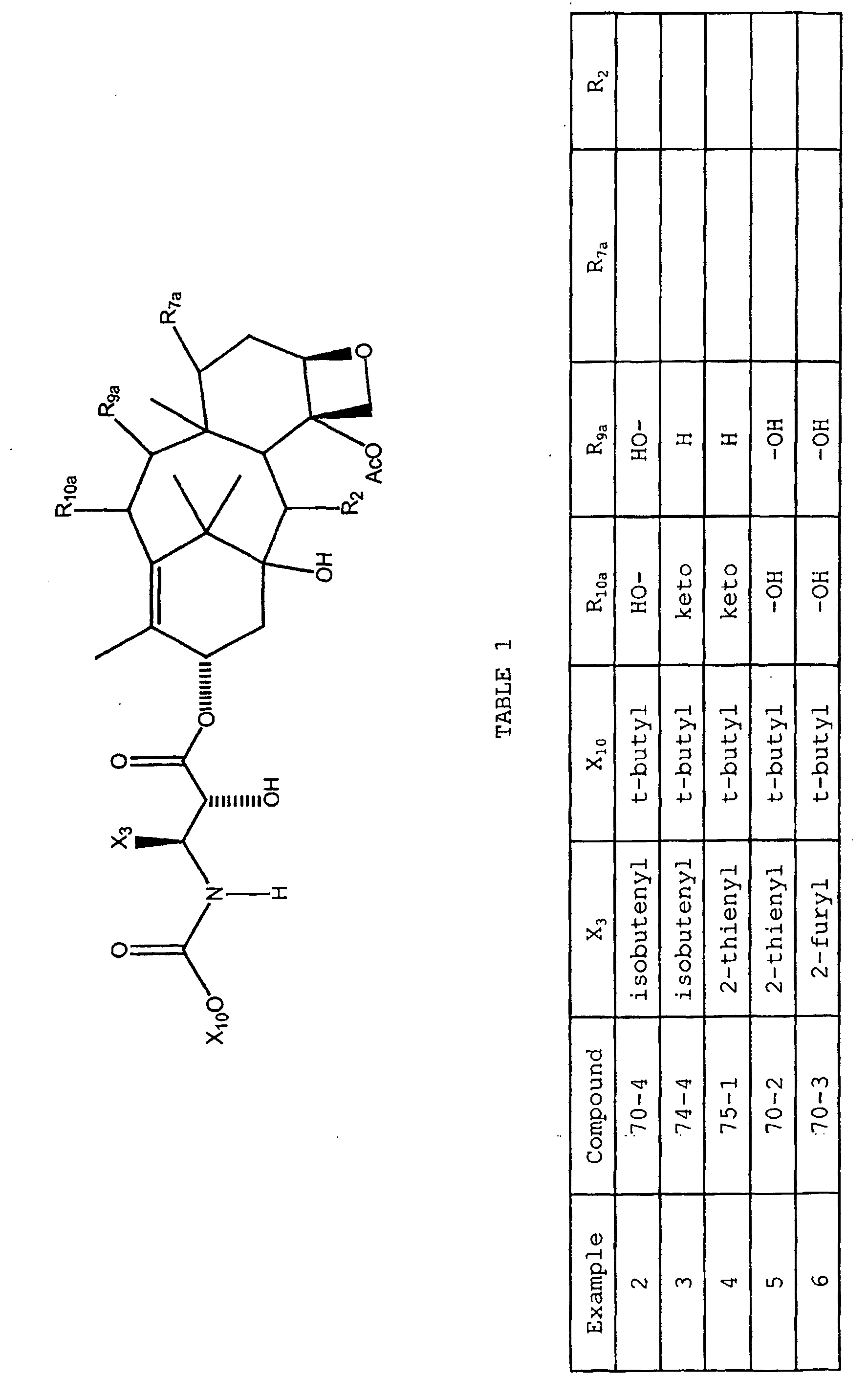

### EXAMPLE

The taxanes of the preceding Examples were evaluated in in vitro cytotoxicity activity against human colon carcinoma cells HCT-116. Cytotoxicity was assessed in HCT116 human colon carcinoma cells by XTT (2,3-bis(2-methoxy-4-nitro-5-sulfophenyl)-5-[(phenylamino)carbonyl]-2H-tetrazolium hydroxide) assay (Scudiero et al, "Evaluation of a soluble tetrazolium/formazan assay for cell growth and drug sensitivity in culture using human and other tumor cell lines", Cancer Res. 48:4827-4833, 1988). Cells were plated at 4000 cells/well in 96 well microtiter plates and 24 hours later drugs were added and serial diluted. The cells were incubated at 37°C for 72 hours at which time the tetrazolium dye, XTT, was added. A dehydrogenase enzyme in live cells reduces the XTT to a form that absorbs light at 450 nm which can be quantitated spectrophotometrically. The greater the absorbance the greater the number of live cells. The results are expressed as an IC₅₀ which is the drug concentration required to inhibit cell proliferation (i.e. absorbance at 450 nm) to 50% of that of untreated control cells.

All compounds had an IC₅₀ of less than 0.1, indicating that they are cytotoxically active.

## Claims

1. A taxane derivative having the formula: 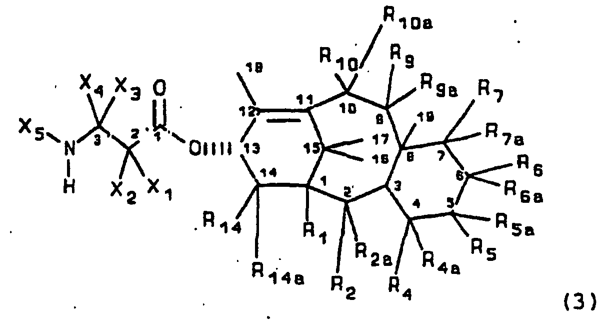 wherein
X₁ is -OX₆, -SX₇, or -NX₈X₉;
X₂ is hydrogen, alkyl, alkenyl, alkynyl, aryl,
or
heteroaryl;
X₃ and X₄ are independently hydrogen, alkyl, alkenyl, alkynyl, or heteroaryl;
X₅ is -COOX₁₀;
X₆ is hydrogen;
X₇ is alkyl, alkenyl, alkynyl, aryl, heteroaryl, or sulfhydryl protecting group;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is t-butyl;
R₁ is hydrogen, hydroxy, protected hydroxy or together with R₁₄ forms a carbonate;
R₂ is hydrogen, hydroxy, -OCOR₃₁ or together with R₂ₐ forms an oxo;
R₂ₐ is hydrogen or taken together with R₂ forms an oxo or;
R₄ is hydrogen, together with R₄ₐ forms an oxo, oxirane or methylene, or together with R₅ₐ and the carbon atoms to which they are attached form an oxetane ring;
R₄ₐ is hydrogen, alkyl, alkenyl, alkynyl, aryl, heteroaryl, cyano, hydroxy, -OCOR₃₀, or together with R₄ forms an oxo, oxirane or methylene;
R₅ is hydrogen or together with R₅ₐ forms an oxo;
R₅ₐ is hydrogen, hydroxy, acyloxy, together with R₅ forms an oxo, or together with R₄ and the carbon atoms to which they are attached form an oxetane ring;
R₆ is hydrogen, alkyl, alkenyl, alkynyl, aryl, or heteroaryl, hydroxy, or together with R₆ₐ forms an oxo;
R₆ₐ is hydrogen, alkyl, alkenyl, alkynyl, aryl, or heteroaryl, hydroxy, or together with R₆ forms an oxo;
R₇ is hydrogen or together with R₇ₐ forms an oxo;
R₇ₐ is hydroxy, or together with R₇ forms an oxo;
R₉ is hydrogen;
R₉ₐ is hydrogen, hydroxy, or acyloxy;
R₁₀ is hydrogen or together with R₁₀ₐ forms an oxo ;
R₁₀ₐ is hydrogen, -OCOR₂₉, or hydroxy, or together with R₁₀ forms an oxo;
R₁₄ is hydrogen, alkyl, alkenyl, alkynyl, aryl, heteroaryl, hydroxy, or together with R₁ forms a carbonate;
R₁₄ₐ is hydrogen, alkyl, alkenyl, alkynyl, aryl, or heteroaryl; and
R₂₉, R₃₀, and R₃₁ are independently hydrogen, alkyl, alkenyl, alkynyl, monocyclic aryl or monocyclic heteroaryl.

2. A taxane derivative according to claim 1, wherein X₄ is hydrogen, and X₃ is isopropyl, cyclopropyl, n-butyl, t-butyl, amyl, cyclobutyl, cyclohexyl, furyl, thienyl, pyridyl or the substituted analogs thereof.

3. A taxane derivative according to claim 1, wherein R₂ₐ is hydrogen and R₂ is hydroxy or -OCOR₃₁ wherein R₃₁ is hydrogen, alkyl or selected from the group comprising 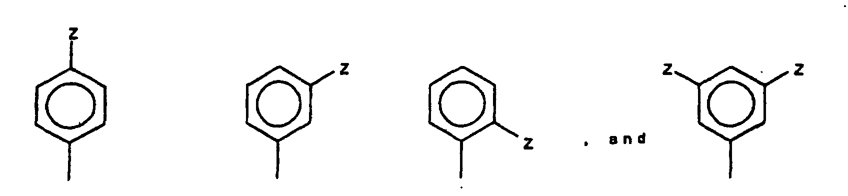 and Z is alkyl, hydroxy, alkoxy, halogen, or trifluoromethyl.

4. A taxane derivative according to claim 1, wherein R₄ together with R₅ₐ and the carbons to which they are attached form an oxetane ring, R₂ₐ is hydrogen and R₂ is hydroxy or -OCOR₃₁ wherein R₃₁ is hydrogen, alkyl or selected from the group comprising 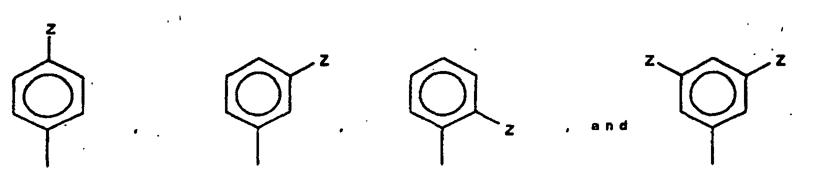 and Z is alkyl, hydroxy, alkoxy, halogen, or trifluoromethyl.

5. A taxane derivative according to claim 1, wherein R₉ is hydrogen and R₉ₐ is hydrogen, hydroxy or acetoxy.

6. A taxane derivative according to claim 1, wherein R₁₀ₐ is hydrogen, together with R₁₀ forms an oxo or is other than hydroxy and acetoxy.

7. A taxane derivative according to claim 1, wherein X₁ is -OH, X₂ is hydrogen, X₃ is isobutenyl, X₄ is hydrogen, R₁ is -OH, R₂ is benzoyloxy, R₂ₐ, R₅, R₆, R₆ₐ, R₇, R₁₀, R₁₄ and R₁₄ₐ are each hydrogen, R₄ together with R₅ₐ and the carbon atoms to which they are attached form an oxetane ring, R₄ₐ is acetoxy, and R₇ₐ, R₉ₐ and R₁₀ₐ are each -OH.

8. A taxane derivative according to claim 1, wherein X₁ is -OH, X₂ is hydrogen, X₃ is isobutenyl, X₄ is hydrogen, R₁ is -OH, R₂ is benzoyloxy, R₂ₐ, R₅, R₆, R₆ₐ, R₇, R₁₄ and R₁₄ₐ are each hydrogen, R₄ together with R₅ₐ and the carbon atoms to which they are attached form an oxetane ring, R₄ₐ is acetoxy, R₇ₐ and R₉ₐ are each -OH, and R₁₀ together with R₁₀ₐ forms an oxo.

9. A taxane derivative according to claim 1, wherein X₁ is -OH, X₂ is hydrogen, X₃ is 2-thienyl, X₄ is hydrogen, R₁ is -OH, R₂ is benzoyloxy, R₂ₐ, R₅, R₆, R₆ₐ, R₇, R₁₄ and R₁₄ₐ are each hydrogen, R₄ together with R₅ₐ and the carbon atoms to which they are attached form an oxetane ring, R₄ₐ is acetoxy, R₇ₐ and R₉ₐ are each -OH, and R₁₀ together with R₁₀ₐ forms an oxo.

10. A taxane derivative according to claim 1, wherein X₁ is -OH, X₂ is hydrogen, X₃ is 2-thienyl, X₄ is hydrogen, R₁ is -OH, R₂ is benzoyloxy, R₂ₐ, R₅, R₆, R₆ₐ, R₇, R₁₀, R₁₄ and R₁₄ₐ are each hydrogen, R₄ together with R₅ₐ and the carbon atoms to which they are attached form an oxetane ring, R₄ₐ is acetoxy, and R₇ₐ, R₉ₐ and R₁₀ₐ are each -OH.

11. A taxane derivative according to claim 1, wherein X₁ is -OH, X₂ is hydrogen, X₃ is 2-furyl, X₄ is hydrogen, R₁ is -OH, R₂ is benzoyloxy, R₂ₐ, R₅, R₆, R₆ₐ, R₇, R₁₀, R₁₄ and R₁₄ₐ are each hydrogen, R₄ together with R₅ₐ and the carbon atoms to which they are attached form an oxetane ring, R₄ₐ is acetoxy, and R₇ₐ, R₉ₐ and R₁₀ₐ are each -OH.

## Patentansprüche

1. Taxanderivat mit der Formel 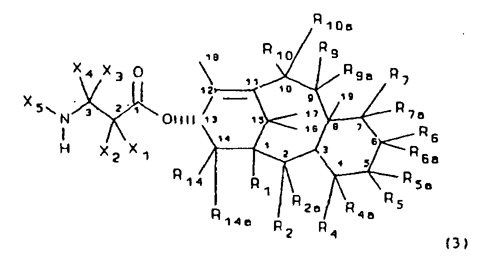 worin
X₁ -OX₆, -SX₇ oder -NX₈X₉ ist,
X₂ Wasserstoff, Alkyl, Alkenyl, Alkynyl, Aryl oder Heteroaryl ist,
X₃ und X₄ unabhängig Wasserstoff, Alkyl, Alkenyl, Alkynyl oder Heteroaryl sind,
X₅ -COOX₁₀ ist,
X₆ Wasserstoff ist,
X₇ Alkyl, Alkenyl, Alkynyl, Aryl, Heteroaryl oder Sulfhydryl-Schutzgruppe ist,
X₈ Wasserstoff, Alkyl, Alkenyl, Alkynyl, Aryl, Heteroaryl oder heterosubstituiertes Alkyl, Alkenyl, Alkynyl, Aryl oder Heteroaryl ist,
X₉ eine Amino-Schutzgruppe ist,
X₁₀ t-Butyl ist,
R₁ Wasserstoff, Hydroxy, geschütztes Hydroxy ist oder zusammen mit R₁₄ ein Carbonat bildet,
R₂ Wasserstoff, Hydroxy, -OCOR₃₁ ist oder zusammen mit R₂ₐ ein Oxo bildet,
R₂ₐ Wasserstoff ist oder zusammen mit R₂ ein Oxo bildet,
R₄ Wasserstoff ist, zusammen mit R₄ₐ ein Oxo, Oxiran oder Methylen bildet oder zusammen mit R₅ₐ und den Kohlenstoffatomen, an denen sie hängen, einen Oxetanring bildet,
R₄ₐ Wasserstoff, Alkyl, Alkenyl, Alkynyl, Aryl, Heteroaryl, Cyano, Hydroxy, -OCOR₃₀ ist oder zusammen mit R₄ ein Oxo, Oxiran oder Methylen bildet,
R₅ Wasserstoff ist oder zusammen mit R₅ₐ ein Oxo bildet
R₅ₐ Wasserstoff, Hydroxy, Acyloxy ist, zusammen mit R₅ ein Oxo bildet oder zusammen mit R₄ und den Kohlenstoffatomen, an denen sie hängen, einen Oxetanring bildet,
R₆ Wasserstoff, Alkyl, Alkenyl, Alkynyl, Aryl, Heteroaryl oder Hydroxy ist oder zusammen mit R₆ₐ ein Oxo bildet,
R₆ₐ Wasserstoff, Alkyl, Alkenyl, Alkynyl, Aryl oder Heteroaryl, Hydroxy ist oder zusammen mit R₆ ein Oxo bildet,
R₇ Wasserstoff ist oder zusammen mit R₇ₐ ein Oxo bildet,
R₇ₐ Hydroxy ist oder zusammen mit R₇ ein Oxo bildet,
R₉ Wasserstoff ist,
R₉ₐ Wasserstoff, Hydroxy oder Acyloxy ist,
R₁₀ Wasserstoff ist oder zusammen mit R₁₀ₐ ein Oxo bildet,
R₁₀ₐ Wasserstoff, -OCOR₂₉ oder Hydroxy ist oder zusammen mit R₁₀ ein Oxo bildet,
R₁₄ Wasserstoff, Alkyl, Alkenyl, Alkynyl, Aryl, Heteroaryl, Hydroxy ist oder zusammen mit R₁ ein Carbonat bildet,
R₁₄ₐ Wasserstoff, Alkyl, Alkenyl, Alkynyl, Aryl oder Heteroaryl ist und
R₂₉ , R₃₀ und R₃₁ unabhängig Wasserstoff, Alkyl, Alkenyl, Alkynyl, monocyclisches Aryl oder monocyclisches Heteroaryl sind.

2. Taxanderivat nach Anspruch 1, bei dem X₁ Wasserstoff ist und X₃ Isopropyl, Cyclopropyl, n-Butyl, t-Butyl, Amyl, Cyclobutyl, Furyl, Thienyl, Pyridyl oder die substituierten Analoga hiervon ist.

3. Taxanderivat nach Anspruch 1, bei dem R₂ₐ Wasserstoff ist und R₂ Hydroxy oder-OCOR₃₁ ist, worin R₃₁ Wasserstoff, Alkyl ist oder aus der aus 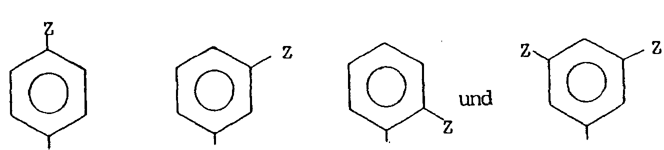 bestehenden Gruppe Gruppe ausgewählt ist und Z Alkyl, Hydroxy, Alkoxy, Halogen oder Trifluormethyl ist.

4. Taxanderivat nach Anspruch 1, bei dem R₄ zusammen mit R₅ₐ und den Kohlenstoffatomen, an denen sie hängen, einen Oxetanring bildet, R₂ₐ Wasserstoff ist und R₂ Hydroxy oder -OCOR₃₁ ist, worin R₃₁ Wasserstoff oder Alkyl ist oder aus der aus 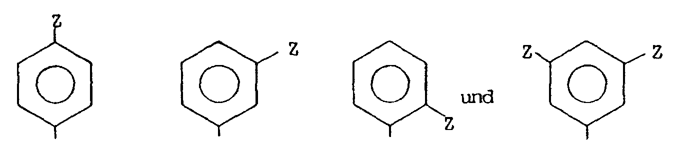 bestehenden Gruppe ausgewählt ist und Z Alkyl, Hydroxy, Alkoxy, Halogen oder Trifluormethyl ist.

5. Taxanderivat nach Anspruch 1, bei dem R₉ Wasserstoff ist und R₉ₐ Wasserstoff, Hydroxy oder Acetoxy ist.

6. Taxanderivat nach Anspruch 1, bei dem R₁₀ₐ Wasserstoff ist, zusammen mit R₁₀ ein Oxo bildet oder von Hydroxy und Acetoxy verschieden ist.

7. Taxanderivat nach Anspruch 1, bei dem X₁ -OH ist, X₂ Wasserstoff ist, X₃ Isobutenyl ist, X₄ Wasserstoff ist, R₁ -OH ist, R₂ Benzoyloxy ist, R₂ₐ , R₅ , R₆ , R₆ₐ , R₇ , R₁₀ , R₁₄ und R₁₄ₐ jeweils Wasserstoff sind, R₄ zusammen mit R₅ₐ und den Kohlenstoffatomen, an denen sie hängen, einen Oxetanring bilden, R₄ₐ Acetoxy ist und R₇ₐ , R₉ₐ und R₁₀ₐ jeweils -OH sind.

8. Taxanderivat nach Anspruch 1, bei dem X₁ -OH ist, X₂ Wasserstoff ist, X₃ Isobutenyl ist, X₄ Wasserstoff ist, R₁ -OH ist, R₂ Benzoyloxy ist, R₂ₐ , R₅ , R₆ , R₆ₐ , R₇ , R₁₄ und R₁₄ₐ jeweils Wasserstoff sind, R₄ zusammen mit R₅ₐ und den Kohlenstoffatomen, an denen sie hängen, einen Oxetanring bilden, R₄ₐ Acetoxy ist, R₇ₐ und R₉ₐ jeweils -OH sind und R₁₀ zusammen mit R₁₀ₐ ein Oxo bildet.

9. Taxanderivat nach Anspruch 1, bei dem X₁ -OH ist, X₂ Wasserstoff ist, X₃ 2-Thienyl ist, X₄ Wasserstoff ist, R₁ -OH ist, R₂ Benzoyloxy ist, R₂ₐ , R₅ , R₆ , R₆ₐ , R₇ , R₁₄ und R₁₄ₐ jeweils Wasserstoff sind, R₄ zusammen mit R₅ₐ und den Kohlenstoffatomen, an denen sie hängen, einen Oxetanring bildet, R₄ₐ Acetoxy ist, R₇ₐ und R₉ₐ jeweils -OH sind und R₁₀ zusammen mit R₁₀ₐ ein Oxo bildet.

10. Taxanderivat nach Anspruch 1, bei dem X₁ -OH ist, X₂ Wasserstoff ist, X₃ 2-Thienyl ist, X₄ Wasserstoff ist, R₁ -OH ist, R₂ Benzoyloxy ist, R₂ₐ , R₅ , R₆ , R₆ₐ , R₇ , R₁₀ , R₁₄ und R₁₄ₐ jeweils Wasserstoff sind, R₄ zusammen mit R₅ₐ und den Kohlenstoffatomen, an denen sie hängen, einen Oxetanring bildet, R₄ₐ Acetoxy ist und R₇ₐ , R₉ₐ und R₁₀ₐ jeweils -OH sind.

11. Taxanderivat nach Anspruch 1, bei dem X₁ -OH ist, X₂ Wasserstoff ist, X₃ 2-Furyl ist, X₄ Wasserstoff ist, R₁ -OH ist, R₂ Benzoyloxy ist, R₂ₐ , R₅ , R₆ , R₆ₐ , R₇ , R₁₀ , R₁₄ und R₁₄ₐ jeweils Wasserstoff sind, R₄ zusammen mit R₅ₐ und den Kohlenstoffatomen, an denen sie hängen, einen Oxetanring bilden, R₄ₐ Acetoxy ist und R₇ₐ , R₉ₐ und R₁₀ₐ jeweils -OH sind.

## Revendications

1. Dérivé de taxane ayant la formule suivante : 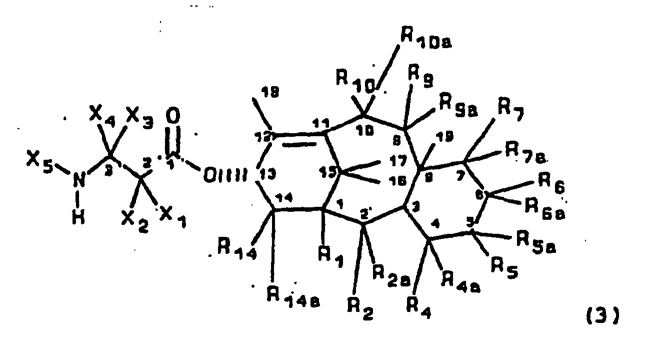 Dans laquelle
X₁ est -OX₆, -SX₇, ou -NX₈X₉
X₂ est un hydrogène, un alkyle, un alcényle, un alcynyle, un aryle ou un hétéroaryle
X₃ et X₄ sont indépendamment un hydrogène, un alkyle, un alcényle, un alcynyle ou un hétéroaryle ;
X₅ est -COOX₁₀
X₆ est un hydrogène
X₇ est un alkyle, un alcényle, un alcynyle, un aryle, un hétéroaryle, ou un groupe de protection de sulfhydryle,
X₈ est un hydrogène, un alkyle, un alcényle, un alcynyle, un aryle, un hétéroaryle, ou un alkyle, alcényle, alcynyle, aryle ou hétéroaryle hétérosubstitués ;
X₉ est un groupe de protection aminé
X₁₀ est un t-butyle
R₁ est un hydrogène, un hydroxy, un hydroxy protégé ou avec R₁₄, il forme un carbonate
R₂ est un hydrogène, un hydroxy, un -OCOR₁₁ ou avec R₂ₐ il forme un oxo ;
R₂ₐ est un hydrogène ou avec R₂, il forme un oxo ou ;
R₄ est un hydrogène, avec R₄ₐ il forme un oxo, un oxirane ou méthylène, ou avec R₅ₐ et les atomes de carbone auxquels ils sont fixés, ils forment un anneau d'oxétane ;
R₄ₐ est un hydrogène, un alkyle, un alcényle, un alcynyle, un aryle, un hétéroaryle, un cyano, un hydroxy, un -OCOR₁₀, ou avec R₄, il forme un oxo, un oxirane ou un méthylène,
R₅ est un hydrogène ou avec R₅ₐ, il forme un oxo
R₅ₐ est un hydrogène, un hydroxy, un acyloxy, avec R₅, il forme un oxo, ou avec R₄, et les atomes de carbone auxquels ils sont fixés, ils forment un anneau d'oxétane
R₆ est un hydrogène, un alkyle, un alcényle, un alcynyle, un aryle, ou hétéroaryle, un hydroxy, ou avec R₆ₐ, il forme un oxo ;
R₆ₐ est un hydrogène, un alkyle, un alcényle, un alcynyle, un aryle, ou hétéroaryle, un hydroxy ou avec R₆, il forme un oxo ;
R₇ est un hydrogène, ou avec R₇ₐ il forme un oxo
R₇ₐ est un hydroxy, ou avec R₇, il forme un oxo
R₉ est un hydrogène ;
R₉ₐ est un hydrogène, un hydroxy ou un acyloxy
R₁₀ est un hydrogène, ou avec R₁₀ₐ, il forme un oxo
R₁₀ₐ est un hydrogène, un -OCOR₂₉, ou un hydroxy ou avec R₁₀, il forme un oxo.
R₁₄ est un hydrogène, un alkyle, un alcényle, un alcynyle, un aryle, un hétéroaryle, un hydroxy ou avec R₁, il forme un carbonate ;
R₁₄ₐ est un hydrogène, un alkyle, un alcényle, un alcynyle, un aryle ou un hétéroaryle, et
R₂₅, R₃₀ et R₃₁ sont indépendamment un hydrogène, un alkyle, un alcényle, un alcynyle, un aryle monocyclique ou un hétéroaryle monocyclique.

2. Dérivé de taxane selon la revendication 1, dans lequel X₄ est un hydrogène et X₃ est un isopropyle, un cyclopropyle, un n-butyle, un t-butyle, un amyle, un cyclobutyle, un cyclohexyle, un furyle, un thiényle, un pyridyle, ou leurs analogues substitués.

3. Dérivé de taxane selon la revendication 1, dans lequel R₂ₐ est un hydrogène et R₂ est un hydroxy ou -OCOR₃₁, dans lequel R₃₁ est un hydrogène, un alkyle ou est choisi dans le groupe comprenant 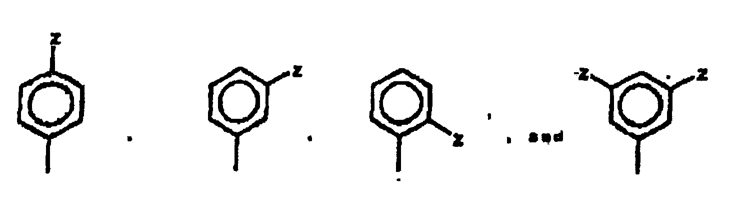 et Z est un alkyle, un hydroxy, un alcoxy, un halogène ou un trifluorométhyle.

4. Dérivé de taxane selon la revendication 1, dans lequel R₄ avec R₅ₐ et les atomes de carbone auxquels ils sont fixés forment un anneau d'oxétane, R₂ₐ est un hydrogène et R₂ est un hydroxy ou un -OCOR₃₁ dans lequel R₃₁ est un hydrogène, un alkyle ou est choisi dans le groupe comprenant 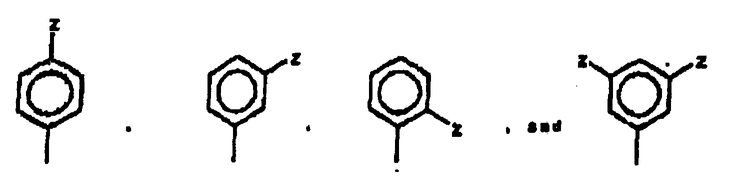 Et Z est un alkyle, un hydroxy, un alcoxy, un halogène ou un trifluorométhyle.

5. Dérivé de taxane selon la revendication 1, dans lequel R₉ est un hydrogène, et R₉ₐ est un hydrogène, un hydroxy ou un acétoxy.

6. Dérivé de taxane selon la revendication 1, dans lequel R₁₀ₐ est un hydrogène, avec R₁₀, il forme un oxo ou est différent de l'hydroxy et de l'acétoxy.

7. Dérivé de taxane selon la revendication 1, dans lequel X₁ est un -OH, X₂ est un hydrogène, X₃ est un isobutényle, X₄ est un hydrogène, R₁ est un -OH, R₂ est un benzoyloxy, R₂ₐ, R₅, R₆, R₆ₐ, R₇, R₁₀, R₁₄ et R₁₄ₐ sont chacun un hydrogène, R₄ avec R₅ₐ et les atomes de carbone auxquels ils sont fixés forment un anneau d'oxétane, R₄ₐ est un acétoxy, et R₇ₐ, R₅ₐ et R₁₀ₐ sont chacun un -OH.

8. Dérivé de taxane selon la revendication 1, dans lequel X₁ est un -OH, X₂ est un hydrogène, X₃ est un isobutényle, X₄ est un hydrogène, R₁ est un -OH, R₂ est un benzoyloxy, R₂ₐ, R₅, R₆, R₆ₐ, R₇, R₁₄ et R₁₄ₐ sont chacun un hydrogène, R₄ avec R₅ₐ et les atomes de carbone auxquels ils sont fixés forment un anneau d'oxétane, R₄ₐ est un acétoxy, R₇ₐ et R₅ₐ sont chacun un -OH, et R₁₀ avec R₁₀ₐ forment un oxo.

9. Dérivé de taxane selon la revendication 1, dans lequel X₁ est un -OH, X₂ est un hydrogène, X₃ est un 2-thiényle, X₄ est un hydrogène, R₁ est un -OH, R₂ est un benzoyloxy, R₂ₐ, R₅, R₆, R₆ₐ, R₇, R₁₄, et R₁₄ₐ sont chacun un hydrogène, R₄ avec R₅ₐ et les atomes de carbone auxquels ils sont fixés forment un anneau d'oxétane, R₄ₐ est un acétoxy, R₇ₐ et R₉ₐ sont chacun un -OH, et R₁₀ avec R₁₀ₐ forment un oxo.

10. Dérivé de taxane selon la revendication 1, dans lequel X₁ est un -OH, X₂ est un hydrogène, X₃ est un 2-thiényle, X₄ est un hydrogène, R₁ est un -OH, R₂ est un benzoyloxy, R₂ₐ, R₅, R₆, R₆ₐ, R₇, R₁₀, R₁₄, et R₁₄ₐ sont chacun un hydrogène, R₄ avec R₅ₐ et les atomes de carbone auxquels ils sont fixés forment un anneau d'oxétane, R₄ₐ est un acétoxy, et R₇ₐ, R₉ₐ et R₁₀ₐ sont chacun un -OH.

11. Dérivé de taxane selon la revendication 1, dans lequel X₁ est un -OH, X₂ est un hydrogène, X₃ est un 2-furyle, X₄ est un hydrogène, R₁ est un -OH, R₂ est un benzoyloxy, R₂ₐ, R₅, R₆, R₆ₐ, R₇, R₁₀, R₁₄ et R₁₄ₐ sont chacun un hydrogène, R₄ avec R₅ₐ et les atomes de carbone auxquels ils sont liés forment un anneau d'oxétane, R₄ₐ est un acétoxy et R₇ₐ, R₉ₐ et R₁₀ₐ sont chacun un -OH.
